# EUROPEAN PATENT SPECIFICATION

(11) **EP 2 420 575 B1**
(45) Date of publication and mention of the grant of the patent: **30.03.2016**
(21) Application number: 10764682.0
(22) Date of filing: 16.04.2010
(51) Int. Cl.: C12Q 1/68, G01N 33/68

(54) **MARKER FOR PROGNOSIS OF LIVER CANCER**
MARKER FÜR LEBERKREBSPROGNOSE
MARQUEUR PRONOSTIQUE DU CANCER DU FOIE

(30) Priority: 17.04.2009 KR 20090033638
(43) Date of publication of application: 22.02.2012
(73) Proprietor: CBS Bioscience, Co., Ltd, Daejeon 305-343 (KR); Shim, Youngtack, Seoul 135-110 (KR)
(72) Inventor: PARK, Jin young, Seoul 139-220 (KR); HONG, Seok joo, Daejeon 302-751 (KR); KIM, Jongmin, Seoul 138-225 (KR)
(74) Representative: Lux, Berthold
(86) International application number: PCT/KR2010/002373
(87) International publication number: WO 2010/120143

(56) References cited:
- WO-A1-2007/063118
- WO-A2-02/29103
- KR-A- 20040 101 992
- US-A1- 2007 202 496
- US-A1- 2008 112 956
- MATÍAS A AVILA ET AL: "Reduced mRNA abundance of the main enzymes involved in methionine metabolism in human liver cirrhosis and hepatocellular carcinoma", JOURNAL OF HEPATOLOGY, vol. 33, no. 6, 1 December 2000 (2000-12-01), pages 907-914, XP055046841, ISSN: 0168-8278, DOI: 10.1016/S0168-8278(00)80122-1
- VIKTOR KOZICH ET AL: "Screening for mutations by expressing patient cDNA segments inE. coli: Homocystinuria due to cystathionine [beta]-synthase deficiency", HUMAN MUTATION, vol. 1, no. 2, 1 January 1992 (1992-01-01) , pages 113-123, XP055046983, ISSN: 1059-7794, DOI: 10.1002/humu.1380010206
- A. PRUDOVA: "S-adenosylmethionine stabilizes cystathionine beta-synthase and modulates redox capacity", PROCEEDINGS OF THE NATIONAL ACADEMY OF SCIENCES, vol. 103, no. 17, 25 April 2006 (2006-04-25), pages 6489-6494, XP055046843, ISSN: 0027-8424, DOI: 10.1073/pnas.0509531103
- DATABASE WPI Week 2008 Thomson Scientific, London, GB; AN 2008-A48215 XP002688778, CHIBA T; TANIGUCHI H: "Identifying hepatic cancer and determining disease stage or estimating prognosis, involves measuring expression of specific gene in blood, hepatic cancer cell and hepatic cancer tissue", & WO 2007/132883 A (UNIV YOKOHAMA CITY) 22 November 2007 (2007-11-22)
- OKABE H ET AL: "Genome-wide analysis of gene expression in human hepatocellular carcinomas using cDNA microarray: Identification of genes involved in viral carcinogenesis and tumor progression", CANCER RESEARCH, AMERICAN ASSOCIATION FOR CANCER RESEARCH, US, vol. 61, no. 5, 1 March 2001 (2001-03-01), pages 2129-2137, XP002235524, ISSN: 0008-5472
- JU-SEOG LEE ET AL: "Genome-scale profiling of gene expression in hepatocellular carcinoma: Classification, survival prediction, and identification of therapeutic targets", GASTROENTEROLOGY, vol. 127, no. 5, 1 November 2004 (2004-11-01), pages S51-S55, XP55046826, ISSN: 0016-5085, DOI: 10.1053/j.gastro.2004.09.015
- MANN ET AL: "Prognostic molecular markers in hepatocellular carcinoma: A systematic review", EUROPEAN JOURNAL OF CANCER, PERGAMON PRESS, OXFORD, GB, vol. 43, no. 6, 28 March 2007 (2007-03-28) , pages 979-992, XP022003596, ISSN: 0959-8049, DOI: 10.1016/J.EJCA.2007.01.004
- KIM: "Expression of cystathionine [beta]-synthase is downregulated in hepatocellular carcinoma and associated with poor prognosis", ONCOLOGY REPORTS, vol. 21, no. 6, 6 May 2009 (2009-05-06), XP055046838, ISSN: 1021-335X, DOI: 10.3892/or_00000373
- DATABASE GENBANK 15 February 2009 'Homo sapiens cystathionine-beta-synthase (CBS), transcript variant 1, mRNA' Database accession no. NM_000071
- ANNA PRUDOVA ET AL.: 'S-adenosylmethionine stabilizes cystathionine beta-synthase and modulates redox capacity.' PNAS. vol. 103, no. 17, 2006, pages 6489 - 6494

<110> CBSBIOSCIENCE, CO., LTD
<120> MARKERS FOR LIVER CANCER PROGNOSIS
<130> Y09KP-027
<160> 78
<170> KopatentIn 1.71
<210> 1
   <211> 23
   <212> DNA
   <213> Artificial Sequence
<220>
   <223> CBS forward primer
<400> 1
   gttggcaaag tcatctacaa gca 23
<210> 2
   <211> 19
   <212> DNA
   <213> Artificial Sequence
<220>
   <223> CBS reverse primer
<400> 2
   gggcgaagtg gtccatctc 19
<210> 3
   <211> 21
   <212> DNA
   <213> Artificial Sequence
<220>
   <223> CBS probe
<400> 3
   acgctgggca ggctctcgca c 21
<210> 4
   <211> 24
   <212> DNA
   <213> Artificial Sequence
<220>
   <223> NNMT forward primer
<400> 4
   ttgaggtgat ctcgcaaagt tatt 24
<210> 5
   <211> 18
   <212> DNA
   <213> Artificial Sequence
<220>
   <223> NNMT reverse primer
<400> 5
   ctcgccacca gggagaaa 18
<210> 6
   <211> 24
   <212> DNA
   <213> Artificial Sequence
<220>
   <223> NNMT probe
<400> 6
   ccaccatggc caacaacgaa ggac 24
<210> 7
   <211> 22
   <212> DNA
   <213> Artificial Sequence
<220>
   <223> TKT forward primer
<400> 7
   gaggctgtgt ccagtgcagt ag 22
<210> 8
   <211> 22
   <212> DNA
   <213> Artificial Sequence
<220>
   <223> TKT reverse primer
<400> 8
   ccacttcttg gtacccggtt aa 22
<210> 9
   <211> 24
   <212> DNA
   <213> Artificial Sequence
<220>
   <223> TKT probe
<400> 9
   cctggcatca ctgtcaccca cctg 24
<210> 10
   <211> 21
   <212> DNA
   <213> Artificial Sequence
<220>
   <223> BCL2 forward primer
<400> 10
   catgtgtgtg gagagcgtca a 21
<210> 11
   <211> 21
   <212> DNA
   <213> Artificial Sequence
<220>
   <223> BCL2 reverse primer
<400> 11
   gccggttcag gtactcagtc a 21
<210> 12
   <211> 23
   <212> DNA
   <213> Artificial Sequence
<220>
   <223> BCL2 probe
<400> 12
   cctggtggac aacatcgccc tgt 23
<210> 13
   <211> 23
   <212> DNA
   <213> Artificial Sequence
<220>
   <223> FAS forward primer
<400> 13
   aatggtgtca atgaagccaa aat 23
<210> 14
   <211> 25
   <212> DNA
   <213> Artificial Sequence
<220>
   <223> FAS reverse primer
<400> 14
   gtcatacgct tctttctttc catga 25
<210> 15
   <211> 30
   <212> DNA
   <213> Artificial Sequence
<220>
   <223> FAS probe
<400> 15
   tgacaatgtc caagacacag cagaacagaa 30
<210> 16
   <211> 23
   <212> DNA
   <213> Artificial Sequence
<220>
   <223> BAX forward primer
<400> 16
   ggttgtcgcc cttttctact ttg 23
<210> 17
   <211> 18
   <212> DNA
   <213> Artificial Sequence
<220>
   <223> BAX reverse primer
<400> 17
   cagttccggc accttggt 18
<210> 18
   <211> 24
   <212> DNA
   <213> Artificial Sequence
<220>
   <223> BAX probe
<400> 18
   cagcaaactg gtgctcaagg ccct 24
<210> 19
   <211> 24
   <212> DNA
   <213> Artificial Sequence
<220>
   <223> BCL2L1 forward primer
<400> 19
   gattgccttt gttttgatgt ttgt 24
<210> 20
   <211> 22
   <212> DNA
   <213> Artificial Sequence
<220>
   <223> BCL2L1 reverse primer
<400> 20
   ggaaagggaa cccaggttag tg 22
<210> 21
   <211> 29
   <212> DNA
   <213> Artificial Sequence
<220>
   <223> BCL2L1 probe
<400> 21
   cagaattgat cattttcccc ccactctcc 29
<210> 22
   <211> 29
   <212> DNA
   <213> Artificial Sequence
<220>
   <223> AIFM1 forward primer
<400> 22
   tgaagatctc aatgaagtag ccaaactat 29
<210> 23
   <211> 19
   <212> DNA
   <213> Artificial Sequence
<220>
   <223> AIFM1 reverse primer
<400> 23
   ctgcagtggg tttgccaat 19
<210> 24
   <211> 28
   <212> DNA
   <213> Artificial Sequence
<220>
   <223> AIFM1 probe
<400> 24
   caacattcat gaagactgaa gccccaca 28
<210> 25
   <211> 25
   <212> DNA
   <213> Artificial Sequence
<220>
   <223> CASP8 forward primer
<400> 25
   ccttctgatt gatggtgcta ttttg 25
<210> 26
   <211> 18
   <212> DNA
   <213> Artificial Sequence
<220>
   <223> CASP8 reverse primer
<400> 26
   gcggtgagcc gagatcac 18
<210> 27
   <211> 24
   <212> DNA
   <213> Artificial Sequence
<220>
   <223> CASP8 probe
<400> 27
   cagaatctcg ctctgtcgcc cagg 24
<210> 28
   <211> 23
   <212> DNA
   <213> Artificial Sequence
<220>
   <223> CSE1L forward primer
<400> 28
   gcatgatcct gtaggtcaaa tgg 23
<210> 29
   <211> 22
   <212> DNA
   <213> Artificial Sequence
<220>
   <223> CSE1L reverse primer
<400> 29
   caggcggtag acaacttgtg aa 22
<210> 30
   <211> 30
   <212> DNA
   <213> Artificial Sequence
<220>
   <223> CSE1L probe
<400> 30
   aataacccca aaattcacct ggcacagtca 30
<210> 31
   <211> 22
   <212> DNA
   <213> Artificial Sequence
<220>
   <223> XIAP forward primer
<400> 31
   gacaggccat ctgagacaca tg 22
<210> 32
   <211> 23
   <212> DNA
   <213> Artificial Sequence
<220>
   <223> XIAP reverse primer
<400> 32
   agcatagtct ggccagttct gaa 23
<210> 33
   <211> 28
   <212> DNA
   <213> Artificial Sequence
<220>
   <223> XIAP probe
<400> 33
   agacaccata tacccgagga accctgcc 28
<210> 34
   <211> 24
   <212> DNA
   <213> Artificial Sequence
<220>
   <223> DIABLO forward primer
<400> 34
   aatcacattc agctggtgaa actg 24
<210> 35
   <211> 20
   <212> DNA
   <213> Artificial Sequence
<220>
   <223> DIABLO reverse primer
<400> 35
   tgccagcttg gtttctgctt 20
<210> 36
   <211> 23
   <212> DNA
   <213> Artificial Sequence
<220>
   <223> DIABLO probe
<400> 36
   aagaggtgca ccagctctcc egg 23
<210> 37
   <211> 21
   <212> DNA
   <213> Artificial Sequence
<220>
   <223> AKT1 forward primer
<400> 37
   tctcgggtgc atttgagaga a 21
<210> 38
   <211> 22
   <212> DNA
   <213> Artificial Sequence
<220>
   <223> AKT1 reverse primer
<400> 38
   acagcacaaa aacgtctttc ca 22
<210> 39
   <211> 22
   <212> DNA
   <213> Artificial Sequence
<220>
   <223> AKT1 probe
<400> 39
   ccacgctgtc ctctcgagcc ca 22
<210> 40
   <211> 18
   <212> DNA
   <213> Artificial Sequence
<220>
   <223> PTEN forward primer
<400> 40
   tggcggaact tgcaatcc 18
<210> 41
   <211> 24
   <212> DNA
   <213> Artificial Sequence
<220>
   <223> PTEN reverse primer
<400> 41
   gctgagggaa ctcaaagtac atga 24
<210> 42
   <211> 29
   <212> DNA
   <213> Artificial Sequence
<220>
   <223> PTEN probe
<400> 42
   atattcctcc aattcaggac ccacacgac 29
<210> 43
   <211> 21
   <212> DNA
   <213> Artificial Sequence
<220>
   <223> FRAP1 forward primer
<400> 43
   aggccgcatt gtctctatca a 21
<210> 44
   <211> 24
   <212> DNA
   <213> Artificial Sequence
<220>
   <223> FRAP1 reverse primer
<400> 44
   gcagtaaatg caggtagtca tcca 24
<210> 45
   <211> 22
   <212> DNA
   <213> Artificial Sequence
<220>
   <223> FRAP1 probe
<400> 45
   tgcaatccag ctgtttggcg cc 22
<210> 46
   <211> 23
   <212> DNA
   <213> Artificial Sequence
<220>
   <223> PRKAA1 forward primer
<400> 46
   ggcagttgcc taccatctca taa 23
<210> 47
   <211> 20
   <212> DNA
   <213> Artificial Sequence
<220>
   <223> PRKAA1 reverse primer
<400> 47
   gccgagtcag gtgatgatca 20
<210> 48
   <211> 27
   <212> DNA
   <213> Artificial Sequence
<220>
   <223> PRKAA1 probe
<400> 48
   tctatttggc gacaagccca cctgatt 27
<210> 49
   <211> 23
   <212> DNA
   <213> Artificial Sequence
<220>
   <223> ATG3 forward primer
<400> 49
   ccattgaaaa tcaccctcat ctg 23
<210> 50
   <211> 19
   <212> DNA
   <213> Artificial Sequence
<220>
   <223> ATG3 reverse primer
<400> 50
   cacctcagca tgcctgcat 19
<210> 51
   <211> 27
   <212> DNA
   <213> Artificial Sequence
<220>
   <223> ATG3 probe
<400> 51
   caccacctcc catgtgttca gttcacc 27
<210> 52
   <211> 22
   <212> DNA
   <213> Artificial Sequence
<220>
   <223> ATG12 forward primer
<400> 52
   cgacagcttc gatttgaatg ac 22
<210> 53
   <211> 17
   <212> DNA
   <213> Artificial Sequence
<220>
   <223> ATG12 reverse primer
<400> 53
   gggagcgtcg caaagga 17
<210> 54
   <211> 25
   <212> DNA
   <213> Artificial Sequence
<220>
   <223> ATG12 probe
<400> 54
   tgtaattgcg tccccctact ccggc 25
<210> 55
   <211> 22
   <212> DNA
   <213> Artificial Sequence
<220>
   <223> ATG5 forward primer
<400> 55
   tttcctccac tgccatcatt aa 22
<210> 56
   <211> 20
   <212> DNA
   <213> Artificial Sequence
<220>
   <223> ATG5 reverse primer
<400> 56
   ggccaaaggt ttcagcttca 20
<210> 57
   <211> 29
   <212> DNA
   <213> Artificial Sequence
<220>
   <223> ATG5 probe
<400> 57
   cctcagctgt gacatgaaag acttaccgg 29
<210> 58
   <211> 19
   <212> DNA
   <213> Artificial Sequence
<220>
   <223> ATG7 forward primer
<400> 58
   cggatgaatg agcctccaa 19
<210> 59
   <211> 24
   <212> DNA
   <213> Artificial Sequence
<220>
   <223> ATG7 reverse primer
<400> 59
   ggacattatc aaaccgtgaa agaa 24
<210> 60
   <211> 25
   <212> DNA
   <213> Artificial Sequence
<220>
   <223> ATG7 probe
<400> 60
   tcttgggctt gtgcctcacc agatc 25
<210> 61
   <211> 19
   <212> DNA
   <213> Artificial Sequence
<220>
   <223> BNIP3 forward primer
<400> 61
   ttccccccaa ggagttcct 19
<210> 62
   <211> 19
   <212> DNA
   <213> Artificial Sequence
<220>
   <223> BNIP3 reverse primer
<400> 62
   cgctcgtgtt cctcatgct 19
<210> 63
   <211> 19
   <212> DNA
   <213> Artificial Sequence
<220>
   <223> BNIP3 probe
<400> 63
   acccgaagcg cacggccac 19
<210> 64
   <211> 22
   <212> DNA
   <213> Artificial Sequence
<220>
   <223> DRAM forward primer
<400> 64
   cagccgcctt cattatctcc ta 22
<210> 65
   <211> 21
   <212> DNA
   <213> Artificial Sequence
<220>
   <223> DRAM reverse primer
<400> 65
   tggaggtgtt gttcccgtat c 21
<210> 66
   <211> 21
   <212> DNA
   <213> Artificial Sequence
<220>
   <223> DRAM probe
<400> 66
   tgctctccgg gcacgtcaac c 21
<210> 67
   <211> 25
   <212> DNA
   <213> Artificial Sequence
<220>
   <223> LAMP1 forward primer
<400> 67
   tgcactcaga tttaagcctt acaaa 25
<210> 68
   <211> 21
   <212> DNA
   <213> Artificial Sequence
<220>
   <223> LAMP1 reverse primer
<400> 68
   tcaccacgag tgaccttcat g 21
<210> 69
   <211> 24
   <212> DNA
   <213> Artificial Sequence
<220>
   <223> LAMP1 probe
<400> 69
   aagcctctgg ccgtcacacg tagg 24
<210> 70
   <211> 21
   <212> DNA
   <213> Artificial Sequence
<220>
   <223> LC3 forward primer
<400> 70
   aaccagcaca gcatggtgag t 21
<210> 71
   <211> 22
   <212> DNA
   <213> Artificial Sequence
<220>
   <223> LC3 reverse primer
<400> 71
   cctcgtcttt ctcctgctcg ta 22
<210> 72
   <211> 19
   <212> DNA
   <213> Artificial Sequence
<220>
   <223> LC3 probe
<400> 72
   tccacgccca tcgcggaca 19
<210> 73
   <211> 14
   <212> DNA
   <213> Artificial Sequence
<220>
   <223> ULK1 forward primer
<400> 73
   tcgcggccgc atgt 14
<210> 74
   <211> 23
   <212> DNA
   <213> Artificial Sequence
<220>
   <223> ULK1 reverse primer
<400> 74
   aagggtccag cactatcaag aga 23
<210> 75
   <211> 22
   <212> DNA
   <213> Artificial Sequence
<220>
   <223> ULK1 probe
<400> 75
   agcaggtcct gggcgcctca ac 22
<210> 76
   <211> 24
   <212> DNA
   <213> Artificial Sequence
<220>
   <223> E2F1 forward primer
<400> 76
   ctggaccacc tgatgaatat ctgt 24
<210> 77
   <211> 22
   <212> DNA
   <213> Artificial Sequence
<220>
   <223> E2F1 reverse primer
<400> 77
   caatgctacg aaggtcctga ca 22
<210> 78
   <211> 20
   <212> DNA
   <213> Artificial Sequence
<220>
   <223> E2F1 probe
<400> 78
   cagctgcgcc tgctctccga 20

## Description

### Technical Field

The present disclosure relates to a marker for prognosis of liver cancer, a composition for estimating the prognosis of liver cancer comprising a substance for detecting a change in the expression level of the marker for prognosis of liver cancer, a kit for estimating the prognosis of liver cancer which comprises the composition for estimating liver cancer prognosis, a method for estimating the prognosis of liver cancer using the marker for liver cancer prognosis, and a method for screening a therapeutic agent for liver cancer using the marker for prognosis of liver cancer.

### Background Art

Cancer has the same meaning as malignant tumor. It means a condition where the function of modulating cell proliferation is damaged due to various reasons and thus abnormal cells are not controlled and proliferate excessively to penetrate into the surrounding tissues and organs, forming masses and damaging normal tissues. Cancer arising from various tissues in the human body brings one's life in danger due to its rapid growth, infiltrative (penetrating or spreading) growth, spread (moving far away from its original area), etc.

Among cancers, liver cancer is known as one of the most fatal cancers in the world. In particular, it is reported that at least about five hundred thousand people die of liver cancer every year in Asia and in Sub-Saharan Africa. Liver cancer can be largely classified into hepatocellular carcinoma which arises from the liver cell itself, and metastatic liver cancer which is cancer from other tissues spread to the liver. About at least 90% of liver cancer is hepatocellular carcinoma, and the term liver cancer is generally understood to refer to hepatocellular carcinoma.

Most of the causes of liver cancer are reported to be an acute or chronic infection by hepatitis B virus or hepatitis C virus. However, the molecular mechanism within cells relating to the occurrence and development of liver cancer has not been clarified yet. According to conventional researches, it is reported that in case proto-oncogenes such as various growth factor genes are mutated to oncogenes by various reasons to be over-expressed or over-activated, or in case tumor suppressor genes such as Rb protein or p53 protein are mutated by various reasons to be under-expressed or lose function, this may cause the occurrence and progress of various cancers including liver cancer. In particular, with regard to liver cancer, it has been identified that genes such as modified p53, beta-catenin, AXINI, p21(WAF1/CIP1) and p27 Kip, etc. are related thereto. However, recently, it is recognized that the occurrence and progress of most cancers including liver cancer are not due to specific genes alone, but are due to complex interaction of various genes relating to cell cycle, signal delivery, etc. Thus, it would be necessary to get out of focusing only on the expression or function of individual gene or protein and conduct overall research on various genes or protein.

Prognosis of liver cancer refers to anticipating various conditions of the patient suffering from liver cancer such as possibility of full recovery from liver cancer, possibility of recurrence after treatment, possibility of survival of patient after being diagnosed of liver cancer, etc. This may vary depending on various conditions such as severity of the disease, diagnosis point, treatment progress, etc. Liver cancer can be treated efficiently only when various treatment methods are properly applied according to its prognosis. For example, with regard to patients who are estimated to have good prognosis, it would be necessary to avoid dangerous treatment methods which have a possibility to cause severe side effects to the patients such as aggressive chemical treatment or operations, radiation treatment, and select treatment methods which are relatively moderate, conservative and safe. On the other hand, with regard to patients who are estimated to have bad prognosis, chemical treatment or operations, treatment methods such as radiation treatment should be actively conducted in an attempt to increase the survival period or rate.

According to researches conducted until now, the prognosis of liver cancer that has already progressed is extremely bad, and shows a high fatal rate of dying within 6 months from diagnosis, which leaves an average duration of life of only 4 months. However, liver cancer having a size of less than 3 cm has good prognosis, and is known to have a survival rate of 90% for a year without any particular treatment and after surgery, the survival rate of five years is about 40 ~ 50%. However, it is very difficult to estimate the prognosis of liver cancer patients precisely with prior art technology. In order to estimate prognosis accurately, an analysis method which classifies patients into each risk group is required. However, until now, prognosis has been determined depending only on the clinical pathological liver cancer stage at the time of diagnosis and primary surgical treatment without a means for accurately estimating prognosis of liver cancer. However, unfortunately, the prognosis of each liver cancer patient cannot be precisely determined by the liver cancer stage alone.

CBS protein (cystathionine beta-synthase) is an enzyme converting homocysteine to cystathionine, which is known to act in the transsulfuration pathway and play an important role in *in vivo* methionine metabolism [J Biol Chem. 1994 May 20;269(20):14835-40].

NNMT protein (nicotinamide N-methyltransferase) is an enzyme conducting N-methylation of nicotinamide and pyridine, which is known to be involved in the metabolism of various drugs and xenobiotic compounds [Genomics. 1998 Sep 15;52(3):312-24].

TKT protein (transketolase) is an enzyme performing the core role in the non-oxidative pentose phosphate pathway, and as a member of the group, TKTL1 (transketolase-like 1), TKTL2 (transketolase-like 2) are known [J Biol Chem. 1993 Jan 15;268(2):1397-404].

AIFM1 protein (apoptosis-inducing factor, mitochondrion-associated, 1) is flavoprotein also known as AIF. It is known that if apoptosis occurs, the protein moves to the nucleus, causing chromosome agglomeration and fragmentation, and induces the secretion of cytochrome c and caspase-9 from mitochondria [Nature. 1999 Feb 4;397(6718):441-6].

AKT protein is protein associated with AKT2, AKT3, and is known to play a role in delivering growth factor signals through phosphatidylinositol 3-kinase, i.e., delivering signals of platelet-derived growth factor (PDGF), epidermal growth factor (EGF), insulin and insulin-like growth factor I (IGF-I), etc., and when activated, it is known to phosphorylate the apoptosis-related proteins and inactivate them [Proc Natl Acad Sci USA. 1987 Jul;84(14):5034-7]

ATG3 protein (autophagy related 3 homolog) is involved with the binding between human ATG8 homolog such as GATE-16, GABARAP, MAP-LC3 and lipid. It is reported that mice lacking ATG3 do not form autophagosome, and die within a day after birth [J Biol Chem. 2002 Apr 19;277(16):13739-44. Epub 2002 Feb 1].

ATG5 protein (autophagy related 5 homolog) is involved with autophagy. ATG5 is known to form an ATG12-ATG5 conjugate through the action of ATG7 and ATG10, and this ATG12-ATG5 conjugate moves to the autophagosome membrane to act in the binding of ATG8 and phosphatidylethanolamine. It is reported that mice lacking ATG5 do not form autophagosome and die right after birth, and the cut ATG5 can induce the secretion of cytochrome c and activate caspase in mitochondria [Nature 432:1032-1036 (2004)].

ATG7 protein (autophagy related 7 homolog) is involved with autophagy. It is reported that together with ATG10, ATG7 forms ATG12-ATG5 conjugate, and together with ATG3, it is involved with the binding of ATG8 and phosphatidylethanolamine. Mice lacking ATG7 do not form autophagosome and die right after birth [J Biol Chem. 2001 Jan 19;276(3):1701-6. Epub 2000 Nov 28].

ATG12 protein (autophagy related 12 homolog) is involved with autophagy. It is known that ATG12 forms ATG12-ATG5 conjugate through the action of ATG7 and ATG10, and this ATG12-ATG5 conjugate moves to the autophagosome membrane to act in the binding of ATG8 and phosphatidylethanolamine [J Biol Chem. 1998 Dec 18;273(51):33889-92].

BAX protein (BCL2-associated X protein) is one of the BCL2 protein group, and it is known to promote apoptosis by binding with BCL2, and to be involved in loss of mitochondrial membrane potential and secretion of cytochrome c [Cell. 1993 Aug 27;74(4):609-19].

BCL2 protein (B-cell lymphoma protein 2) is a membrane protein of mitochondria, which inhibits apoptosis. It is known to inhibit secretion of cytochrome c from mitochondria, activate caspase and inhibit apoptosis through binding with apoptosis-activation factor [Proc Natl Acad Sci USA. 1986 Jul;83(14):5214-8].

BCL2L1 protein (Bcl-2-like 1 protein) is a member of the BCL2 protein group, and is known to be placed in the outer membrane of the mitochondria and be involved with sharing mitochondrial membrane channel. It is present in two types of isoforms. The longer form, BCL-XL, is known to inhibit apoptosis, and the shorter form, BCL-XS, is known to promote apoptosis [Cell. 1993 Aug 27;74(4):597-608].

BNIP3 protein (BCL2/adenovirus E1B 19kD-interacting protein 3) can bind with E1B 19 kDa protein and BCL2, and is known to induce apoptosis and autophagy [J Exp Med. 1997 Dec 15;186(12):1975-83].

CASP8 protein (caspase 8, apoptosis-related cysteine protease) is an enzyme that falls within the caspase group, which is a caspase acting at the initial phase of the apoptosis mechanism by FAS. If it is activated through the binding with FADD and proteolytic cleavage, it activates various other caspases [Proc Natl Acad Sci USA. 1996 Dec 10;93(25):14486-91; Biochem J. 1997 Aug 15;326 (Pt 1):1-16]]

CSE1L protein (CSE1 chromosome segregation 1-like) is known to play a role in sending again importin-α from the nucleus to the cytoplasm, and to be involved with apoptosis or cell proliferation [Proc Natl Acad Sci USA. 1995 Oct 24;92(22):10427-31].

DIABLO protein (Direct IAP-binding protein with low pI) is a protein placed in the mitochondria, and if apoptosis occurs, it moves to the cytoplasm to bind with IAP (inhibitor of Apoptosis protein) to help caspase activation [Cell. 2000 Jul 7;102(1):43-53].

DRAM protein (damage-regulated autophagy modulator) is a membrane protein of lysosome, and is known to be involved with autophagy controlled by p53 tumor suppressor and to be essential in apoptosis controlled by p53 [Cell. 2006 Jul 14;126(1):121-34].

E2F protein (E2F transcription factor 1) is a member of a transcription factor of E2F group, and is known to be closely involved with controlling cell cycle and tumor suppressor, and to promote cell growth by binding to Rb protein or induce apoptosis relating to p53 [Gene. 1996 Sep 16;173(2):163-9].

FAS protein (APO-1, CD95, TNFRSF6) is a member of TNF receptor group. It is known to receive signal of FAS ligand and compose DISC (death-inducing signaling complex) together with FADD (Fas-associated death domain protein), caspase 8, and caspase 10 to induce apoptosis [J Biol Chem. 1992 May 25;267(15):10709-15].

FRAP1 protein (Mammalian target of rapamycin) is also known as mTOR. It is known to mediate reactions relating to stress such as DNA damage within cells, nutrition depletion, etc., and the TORC2 composite comprising FRAP1 is known to be the target of cell cycle arrest and immunosuppressive action of FKBP12-rapamycin composite [Nature. 1994 Jun 30;369(6483):756-8].

LAMP1 protein (lysosomal-associated membrane protein 1) is also known as CD107a antigen. It is a type of membrane glycoprotein, and appears to be involved with spread of cancer cells [J Biol Chem. 1990 May 5;265(13):7548-51].

LC3 protein (MAP1 light chain 3-like protein 1) is micro-tubule associated protein and is involved with the interaction between micro-tubule and cytoskeleton. LC3 protein is a yeast ATG8 homolog, and is known to bind with phosphatidylethanolamine by the action of various autophagy proteins and compose autophagosome [J Biol Chem. 1994 Apr 15;269(15):11492-7].

PRKAA1 protein (AMP-activated protein kinase, catalytic, alpha-1) is a catalytic subunit of AMPK. This AMPK is a protein that plays the role of detecting the energy level within cells. It is known to activate when the ratio of AMP/ATP increases and to play a role of limiting various biosynthesis reactions [FEBS Lett. 1994 Dec 12;356(1):117-21].

PTEN protein (phosphatase and tensin homolog) is known to be a tumor suppressor, and is inactivated in various types of cancers. It is both a protein phosphatase and a phosphatidylinositol-3,4,5-triphosphate 3-phosphatase at the same time, and is known to deteriorate PI3K-AKT/PKB signal delivery [Nat Genet. 1997 Apr;15(4):356-62].

ULK1 protein (Unc-51-like kinase 1) is a serine/threonine kinase relating to axon growth, and also known as ATG1 homolog. With regard to autophagy, it is known to be phosphorylated by mTOR [Genomics. 1998 Jul 1;51(1):76-85].

XIAP protein (X-linked inhibitor of apoptosis protein) is a member of the IAP group, and among IAPs, it has a strong apoptosis inhibition effect. It is known to inhibit apoptosis and inhibit caspase activity through binding with tumor necrosis factor receptor-associated factor TRAF1, TRAF2 [Nature. 1996 Jan 25;379(6563):349-53].

However, it is not known how the expression level or expression pattern of the proteins changes in detail in the liver tissue, and how the proteins can be used for estimating the prognosis of liver cancer. Also, there is no example of using the protein or genes encoding the protein as marker for prognosis of liver cancer until now.

### Detailed Description

### Technical Subject

It is an object of the present disclosure to provide a biomarker relating to prognosis of liver cancer to estimate prognosis of liver cancer patients easily and precisely, and further to provide an important starting point for developing therapeutic agents for liver cancer. Accordingly, the present disclosure provides a marker for prognosis of liver cancer, a composition for estimating the prognosis of liver cancer comprising a substance for detecting a change in the expression level of the marker for prognosis of liver cancer, a kit for estimating the prognosis of liver cancer which comprises the composition for estimating liver cancer prognosis, a method for estimating the prognosis of liver cancer using the marker for liver cancer prognosis, and a method for screening a therapeutic agent for liver cancer using the marker for prognosis of liver cancer.

### Means for Achieving the Subject

The present inventors compared the degree of gene expression in liver cancer tissues collected from a plurality of patients diagnosed to have liver cancer, and detected genes expressed in a specifically large amount or small amount according to the progress of each patient to discover markers for prognosis of liver cancer that can be used for estimating the prognosis of liver cancer.

The first aspect of the present disclosure relates to a marker for prognosis of liver cancer comprising one or a combination of at least two selected from a group consisting of the following genes:
CBS (cystathionine beta-synthase; NCBI GI: 209862802);
NNMT (nicotinamide N-methyltransferase; NCBI GI: 62953139);
TKT (transketolase; NCBI GI: 205277461);
AIFM1 (Apoptosis-inducing factor 1, mitochondrial; NCBI GI: 22202627);
AKT1 (RAC-alpha serine/threonine-protein kinase; NCBI GI: 62241010);
ATG3 (Autophagy-related protein 3; NCBI GI: 34147490);
ATG5 (Autophagy protein 5; NCBI GI: 92859692);
ATG7 (Autophagy-related protein 7; NCBI GI: 222144225);
ATG12 (Autophagy-related protein 12; NCBI GI: 38261968);
BAX (Apoptosis regulator BAX; NCBI GI: 34335114);
BCL2 (Apoptosis regulator Bcl-2; NCBI GI: 72198188);
BCL2L1 (Apoptosis regulator Bcl-X; NCBI GI: 20336333);
BNIP3 (BCL2/adenovirus E1B 19 kDa protein-interacting protein 3; NCBI GI: 7669480);
CASP8 (Caspase-8; NCBI GI: 122056470);
CSE1L (Exportin-2; NCBI GI: 29029558);
DIABLO (Diablo homolog, mitochondrial; NCBI GI: 218505810);
DRAM (Damage-regulated autophagy modulator; NCBI GI: 110825977);
E2F1 (Transcription factor E2F1; NCBI GI: 168480109);
FAS (Tumor necrosis factor receptor superfamily member 6; NCBI GI: 23510419);
FRAP1 (FKBP12-rapamycin complex-associated protein; NCBI GI: 206725550);
LAMP1 (Lysosome-associated membrane glycoprotein 1; NCBI GI: 112380627);
LC3[MAP1LC3A] (Microtubule-associated proteins 1A/1B light chain 3A; NCBI GI: 31563519);
PRKAA1 (5'-AMP-activated protein kinase catalytic subunit alpha-1; NCBI GI: 94557300);
PTEN (Phosphatidylinositol-3,4,5-trisphosphate 3-phosphatase and dual-specificity protein phosphatase PTEN; NCBI GI: 110224474);
ULK1 (Serine/threonine-protein kinase ULK1; NCBI GI: 225637564); and
XIAP (Baculoviral IAP repeat-containing protein 4; NCBI GI: 32528298).

Prognosis of liver cancer can be estimated from various aspects. However, representatively, it is determined from the aspect of possibility of recurrence, possibility of survival, and possibility of disease-free survival. The research described in the present specification was proceeded by obtaining gene expression profile from liver cancer tissue of a plurality of liver cancer patients, detecting genes presenting a significant difference in expression level by going through statistical data processing process considering the aspects of possibility of recurrence, possibility of survival, possibility of disease-free survival all together, and discovering markers enabling estimation of prognosis of liver cancer.

In the present specification, "marker for prognosis of liver cancer" refers to a gene marker which is the criteria for estimating patients having good or bad prognosis after occurrence of liver cancer. In the present disclosure, the expression level of this marker within liver cancer tissue of a patient with good prognosis is distinctively high or low as compared with the experimentally predetermined standard level. In particular, in the present disclosure, this marker has a significantly low p-value, which value is calculated based on the difference in expression in the liver cancer tissue of patients with good prognosis and bad prognosis. Preferably, the p-value is less than 0.05.

The present disclosure makes an analysis by comparing the gene expression profile of liver cancer tissue of patient with good or bad treatment progress with the standard level, with respect to liver cancer tissues of a plurality of patients who are known to have liver cancer. Thus, the marker confirmed as such can specifically distinguish patients with good or bad prognosis of liver cancer, and accordingly this can be usefully used for estimating prognosis of liver cancer. In addition, considering that the marker confirmed as such is expressed in a specifically large amount or small amount in liver cancer tissue of patients of a specific prognosis, the physiological function of the marker has a possibility to be directly related to the physiological function relating to the occurrence and progress of liver cancer, in particular, the prognosis of liver cancer. Thus, this marker can be usefully used as a target in researching the occurrence and progress of liver cancer or development of a therapeutic agent for liver cancer.

In particular, the marker for prognosis of liver cancer in the first aspect of the present disclosure was discovered based on the statistical analysis of the liver cancer tissue of an unprecedented plurality of patients. Thus, the markers have a greater significance than the conventional markers relating to liver cancer discovered based on gene expression profile of liver cancer tissue of only some patients, have high clinically useful value, and have more accurate estimating power for estimating prognosis of liver cancer.

The second aspect of the present disclosure relates to a composition for estimating the prognosis of liver cancer comprising a substance for specifically detecting the expression level, expression pattern, or both of the marker for prognosis of liver cancer of the first aspect.

Here, the expression level or expression pattern of each marker for prognosis of liver cancer can be detected by general biochemical analysis methods which confirm the level or pattern of mRNA generated by transcription of the corresponding gene. As such an analysis method for confirming the level or pattern of mRNA, there are RT-PCR, competitive RT-PCR, real-time RT-PCR, RNase protection assay, Northern blot, DNA microarray, etc. In addition, any method that is generally carried out in the pertinent art can be used.

Through the above analysis methods, the level or pattern of mRNA in the biological sample of liver cancer patient can be compared with the standard level, and the difference in expression level, expression pattern or both of the marker for prognosis of liver cancer of the present disclosure is detected therefrom. This enables estimating the prognosis of liver cancer patients. In the present specification, "biological sample" means a cell, tissue, etc. separated from human body where the expression level or expression pattern of the marker for prognosis of liver cancer, or existing level or existing pattern of protein encoded by the marker for prognosis of liver cancer can be detected. It can be exemplified by urine, blood, plasma, serum, etc., but is not particularly limited thereto.

The kit for measuring the level or pattern of mRNA by RT-PCR comprises a primer specific to mRNA of the marker for prognosis of liver cancer of the present disclosure. In the present specification, "primer" means a nucleic acid sequence having free 3' hydroxyl group that can complementarily bind to a template and that enables the reverse transcriptase or DNA polymerase to initiate reproduction of template. The primer is a nucleotide having a sequence complementary to the nucleic acid sequence of a specific gene, and a primer of a length of about 7 bp ~ 50 bp, preferably about 10 bp ~ 30 bp, can be used. Other RT-PCR kits may include a test tube or other suitable container, reaction buffer solution, deoxynucleotide (dNTPs), enzyme such as Taq-polymerase and reverse transcriptase, DNAse, RNAse suppresser, DEPC-water, sterile water, etc. according to detailed embodiments. The primer can initiate DNA synthesis in the presence of a reagent for polymerization (i.e., DNA polymerase or reverse transcriptase) and four different nucleoside triphosphates in a suitable buffer solution and temperature. The primer may comprise other base sequences which do not change the basic feature of the primer acting at the initial point of DNA synthesis. The primer can be synthesized chemically using well known methods, and the nucleic acid sequence can be transformed using many means well known in the pertinent art.

The "substance for specifically detecting the expression level, the expression pattern, or both of the marker for prognosis of liver cancer" in the second aspect of the present disclosure can be any substance that can be specifically used for a corresponding marker in a method for analyzing the expression level or expression pattern of the corresponding marker for prognosis of liver cancer, or a combination of at least two of the substances, and it is not limited to a primer for RT-PCR use. The technical characteristic of the second aspect of the present disclosure lies in comparing the expression level or expression pattern of the marker for prognosis of liver cancer in liver cancer tissue of subject liver cancer patients with the standard level and detecting the difference. Thus, any substance that can detect such difference can be used as a "substance for specifically detecting the expression level, the expression pattern, or both of the marker for prognosis of liver cancer" and achieve the target technical effect of the present invention. Also, a person having ordinary skill in the art can select and use a suitable substance referring to common knowledge in the pertinent art according to detailed embodiments.

The third aspect of the present disclosure relates to a composition for estimating the prognosis of liver cancer comprising a substance for specifically detecting the existing level, the existing pattern, or both of the protein encoded by the marker for prognosis of liver cancer of the first aspect.

With regard to the expression level or expression pattern of the marker for prognosis of liver cancer of the present disclosure, in addition to the method of detecting the level or pattern of mRNA according to the expression of each marker as in the second aspect of the present disclosure, the method for detecting an existing level or existing pattern of protein encoded by each marker in the sample to estimate an expression level or expression pattern of each marker can also be used.

The specific detection of existing level or existing pattern of protein encoded by the marker for prognosis of liver cancer in the present disclosure means the process of confirming how much protein encoded by the marker for prognosis of liver cancer of the present disclosure exists in the biological sample and in what pattern it exists. For example, an antibody specifically binding to protein encoded by the marker for prognosis of liver cancer can be used for the process of confirming the existing level or existing pattern. In the present specification, "antibody" means a protein that can specifically bind to epitope of an antigen, and it is a concept including polyclonal antibody, monoclonal antibody and recombinant antibody.

As a method for measuring the existing level or existing pattern of protein using antibody, there are Western blot, ELISA (enzyme linked immunosorbent assay), radioimmunoassay, radioimmunodiffusion, Ouchterlony immunodiffusion, rocket immunoelectrophoresis, tissue immunity staining, immunoprecipitation assay, complement fixation assay, FACS, protein chip, etc. However, in addition to the above, any method commonly used in the pertinent art can be used.

Through the above analysis methods, the formation level or formation pattern of the antigen-antibody composite in a biological sample of subject liver cancer patients can be compared with the standard level, and the existing level or existing pattern of the protein encoded by the marker for prognosis of liver cancer of the present disclosure can be determined therefrom, and finally, the prognosis of liver cancer patients can be estimated.

Here, "antigen-antibody composite" means a composite of protein encoded by a marker for prognosis of liver cancer and an antibody specific thereto. The formation level or formation pattern of the antigen-antibody composite can generally be measured by detecting the size and pattern of the signal of the detection label associated with a secondary antibody. Such detection label can be exemplified by enzyme, fluorescent substance, ligand, luminescent substance, nanoparticles, redox molecules, radio isotope, etc., but are not limited thereto. When enzyme is used as a detection label, as enzymes that can be used, there are β-gluculonidase, β-D-glucosidase, β-D-galacosidase, urease, peroxidase or alkaline phosphatase, acetylcholinesterase, glucose oxidase, hexokinase and GDPase, RNase, glucose oxidase, luciferase, phosphofructokinase, phosphoenolpyruvate carboxylase, aspartate aminotransferase, phosphenolpyruvate decarboxylase, β-latamase, etc., but the enzymes are not limited thereto. When a fluorescent substance is used as a detection label, as fluorescent substances that can be used, there are fluorescein, isothiocyanate, rhodamine, phycoerythrin, phycocyanin, allophycocyanin, o-phthaldehyde, fluorescamine, etc., but the fluorescent substances are not limited thereto. When a ligand is used as a detection label, as a ligand that can be used, there are biotin derivative, etc., but the ligand is not limited thereto. When a luminescent substance is used as a detection label, as luminescent substances that can be used, there are acridium ester, luciferin, luciferase, etc., but the luminescent substances are not limited thereto. When nanoparticles are used as detection label, as nanoparticles that can be used, there are colloidal gold, tinted latex, etc., but the nanoparticles are not limited thereto. When redox molecules are used as detection label, as redox molecules that can be used, there are ferrocene, ruthenium complex, viologen, quinone, Ti ion, Cs ion, diimide, 1,4-benzoquinone, hydroquinone, K4W(CN)8, [Os(bpy)3]2+, [RU(bpy)3]2+, [MO(CN)8]4-, etc., but the redox molecules are not limited thereto. When a radio isotope is used as a detection label, as radio isotopes that can be used, there are 3H, 14C, 32P, 35S, 36C1, 51Cr, 57Co, 58Co, 59Fe, 90Y, 1251, 131I or 186Re, etc., but the radio isotopes are not limited thereto.

In the third aspect of the present disclosure, "a substance for specifically detecting the existing level, the existing pattern, or both of the protein encoded by the marker for prognosis of liver cancer" may be any substance that can be specifically used for the protein encoded by the marker in an analysis method for detecting the existing level or the existing pattern of the protein encoded by the marker for prognosis of liver cancer, but is not necessarily limited to antibodies. The technical characteristics of the third aspect of the present disclosure lie in comparing the existing level or the existing pattern of the protein encoded by the marker for prognosis of liver cancer in biological samples from patients of liver cancer with a baseline and detecting the differences. Thus, any substance may be used as "a substance for specifically detecting the existing level, the existing pattern, or both of the protein encoded by the marker for prognosis of liver cancer" and can achieve technical effects that the present disclosure aims to achieve as long as the substance is capable of detecting such differences. A skilled person in the art can easily select/sort a suitable substance according to specific embodiments based on average knowledge and known technologies in the art.

The fourth aspect of the present disclosure relates to a kit for estimating the prognosis of liver cancer, comprising the composition for estimating the prognosis of liver cancer of the second or third aspect of the present disclosure.

In addition to a substance for specifically detecting the expression level, the expression pattern, or both of the marker for prognosis of liver cancer comprised in the composition for estimating the prognosis of liver cancer, or a substance for specifically detecting the existing level, the existing pattern, or both of the protein encoded by the marker for prognosis of liver cancer, the kit for estimating the prognosis of liver cancer of the present invention may further comprise one or more types of other ingredients, solutions or apparatus, which are suitable for methods of analyzing the expression level or the expression pattern of gene or methods of analyzing the existing level or the existing patter of protein. For example, in the case of the diagnosis kit for detecting the expression level or the expression pattern of gene, the diagnosis kit may comprise essential ingredients required for performing RT-PCR, and in addition to respective primers specific to mRNA of marker genes, this RT-PCR kit may comprise, for example, test tube or other proper container, reaction buffer solution, deoxynucleotide (dNTPs), enzyme such as Taq-polymerase and reverse transcriptase, DNAse, RNAse inhibitor, DEPC-water (DEPCwater), sterile water, gene-specific primer pair that is used as a quantitative control group, according to specific embodiments. Meanwhile, in case where the diagnosis kit is for detecting the existing level or the existing patter of protein, the diagnosis kit may comprise, for example, essential ingredients required for performing ELISA. This ELISA kit may comprise ingredients capable of detecting bound antibodies, for example, a labeled secondary antibody, chromopores, enzyme (for example, enzyme connected to antibody) and its substrate, and an antibody specific to protein of the quantitative control group. Further, according to the specific aspects, the diagnosis kit may comprise DNA microarray or protein microarray.

The fifth aspect of the present disclosure relates to a method for estimating the prognosis of liver cancer comprising step 1, treating biological samples harvested from subject patients of liver cancer with the composition for estimating the prognosis of liver cancer of the second aspect; and step 2, detecting differences in the expression level, the expression pattern, or both of the marker for prognosis of liver cancer of claim 1 by comparing the treatment result of step 1 with a baseline. Further, the fifth aspect of the present disclosure relates to a method for estimating the prognosis of liver cancer comprising step 1, treating biological samples harvested from patients of liver cancer with the composition for estimating the prognosis of liver cancer of the third aspect; and step 2, detecting differences in the existing level, the existing pattern, or both of the protein encoded by the marker for prognosis of liver cancer of claim 1 by comparing the treatment result of step 1 with a baseline.

For example, in the case of detecting the difference in the expression level of the marker for prognosis of liver cancer that is expressed much more in liver cancer tissues from patients whose prognosis of liver cancer is good, if the expression level of the marker in biological samples from the subject patients of liver cancer is higher than a baseline, it can be told that this suggests that the prognosis of liver cancer is relatively good. Meanwhile, for example, in the case of detecting the difference in the expression level of the marker for prognosis of liver cancer that is expressed much more in liver cancer tissues from patients whose prognosis of liver cancer is poor, if the expression level of the marker in biological samples from the subject patients of liver cancer is higher than a baseline, it can be told that this suggests that the prognosis of liver cancer is relatively poor.

Meanwhile, for example, in the case of detecting the difference in the existing level of protein encoded by the marker for prognosis of liver cancer that is expressed much more in liver cancer tissues from patients whose prognosis of liver cancer is good, if the existing level of protein encoded by the marker in biological samples from the subject patients of liver cancer is higher than a baseline, it can be told that this suggests that the prognosis of liver cancer is relatively good. Meanwhile, for example, in the case of detecting the difference in the existing level of protein encoded by the marker for prognosis of liver cancer that is expressed much more in liver cancer tissues from patients whose prognosis of liver cancer is poor, if the existing level of protein encoded by the marker in biological samples from the subject patients of liver cancer is higher than a baseline, it can be told that this suggests that the prognosis of liver cancer is relatively poor.

The sixth aspect of the present disclosure relates to a method for screening a therapeutic agent for liver cancer using the marker for prognosis of liver cancer of the first aspect of the present disclosure.

As the marker for prognosis of liver cancer of the first aspect of the present disclosure shows noticeable difference in expression patterns according to the prognosis of patients of liver cancer, it might be genes directly involved in physiological functions related to occurrence or development of liver cancer, in particular the prognosis. Accordingly, the protein encoded by the marker may be usefully used as a target protein for studying mechanism of occurrence or development of liver cancer or inventing a therapeutic agent for liver cancer. That is, the marker for prognosis of liver cancer of the first aspect of the present disclosure satisfies an important prerequisite for the development of a therapeutic agent for liver cancer, and thus the method for screening a therapeutic agent for liver cancer using this marker is also included in the scope of the present disclosure.

As an example of the sixth aspect of the present disclosure, there is a method for screening a therapeutic agent for liver cancer comprising a step checking whether a test compound promotes or inhibits the expression of the marker for prognosis of liver cancer of the first aspect of the present disclosure.

As the method for screening a therapeutic agent, for example, RT-PCR, competitive RT-PCR, Real-time RT-PCR, RNase protection assay, northern blotting, DNA microarray, SAGE [Serial Analysis of Gene Expression; Velculescu et al, Science 270:484-7], MPSS [Massively Parellel Signature Sequencing; Brenner et al, PNAS. USA. 97, 1665-1670], etc. may be used. In addition to the above methods, various methods known in the art may be used if necessary.

Meanwhile, as another example of the sixth aspect of the present disclosure, there is a method for screening a therapeutic agent for liver cancer comprising step 1, binding a test compound to the protein encoded by the marker for prognosis of liver cancer of the first aspect of the present disclosure; and step 2, checking whether the test compound promotes or inhibits a physiological activity of said protein. As the method for screening a therapeutic agent, for example, a method of fixing the proteinic markers for early diagnosis of liver cancer of the first aspect of the present disclosure to an affinity column and contacting it with samples to purify them [Pandya et al, Virus Res 87: 135-143, 2002], a method of using two-hybrid system, western blotting, a method of High-Throughput Screening [Aviezer et al, J Biomol Screen 6: 171-7, 2001], etc. may be used. In addition to the above methods, various methods known in the art may be used if necessary.

In the sixth aspect of the present disclosure, as test compounds to be used for screening, for example, tissue extracts, expression products of gene library, synthetic compounds, synthetic peptides, natural compounds, etc. may be used, but the test compounds to be used for screening are not limited thereto.

The seventh aspect of the present disclosure relates to an antibody recognizing specifically the protein encoded by the marker for prognosis of liver cancer of the first aspect of the present disclosure.

The antibody recognizing specifically the marker for prognosis of liver cancer of the first aspect of the present disclosure is a representative substance detecting specifically the existing level or the existing pattern of protein encoded by the marker for prognosis of liver cancer, and accordingly can be usefully used for estimating the prognosis of liver cancer. Further, depending on cases, the antibody may specifically promote or inhibit the activity of the protein that plays an important role in occurrence or development of liver cancer, and accordingly may be used as a therapeutic agent for liver cancer.

As the marker for prognosis of liver cancer of the first aspect of the present disclosure has been found, the preparation of polyclonal antibodies, monoclonal antibodies and recombinant antibodies for the protein encoded by the marker for prognosis of liver cancer can be easily carried out by using technologies widely known in the art.

Polyclonal antibodies can be prepared by methods widely known in the art of injecting protein antigen encoded by the marker for prognosis of liver cancer of the first aspect of the present disclosure into animals and collecting blood from the animals to obtain serum comprising antibodies. These polyclonal antibodies can be prepared from various hosts of species of animals, such as goats, rabbits, sheep, monkeys, horses, pigs, cattle, dogs, etc., and the preparation methods are well known in the art.

Monoclonal antibodies can be prepared by using a hybridoma method [Kohler and Milstein (1976) European Jounral of Immunology 6:511-519] or phage antibody library technology [Clackson et al, Nature, 352:624-628, 1991; Marks et al, J. Mol. Biol., 222:58, 1-597, 1991], etc. which are widely known in the art. Conventionally, a hybridoma method uses cells obtained from host animals which are immunologically suitable, such as a mouse, to which protein antigen encoded by the marker for prognosis of liver cancer of the first aspect of the present disclosure has been injected, and a cancer or myeloma cell line as the other population. The tissues obtained from these two populations are fused by a widely known method in the art such as polyethyleneglycol, and then antibody-producing cells are proliferated by a standard tissue cultivation method. After obtaining a homogenous cell population by subcloning according to a limited dilution technique, hybridoma that can produce desired antibodies is cultivated in quantity *in vivo* or *in vitro* according to a known technique. A phage antibody library method is a method of producing monoclonal antibodies by obtaining a gene of the desired antibody, expressing the gene in the form of the fusion protein on the surface of phages, and thereby producing an antibody library *in vitro,* and separating the desired monoclonal antibodies from the library. The monoclonal antibodies prepared by the above method may be separated by using known methods, such as gel electrophoresis, dialysis, salts precipitation, ion exchange chromatography, affinity chromatography, etc.

The antibody of the seventh aspect of the present disclosure comprises functional fragments of an antibody molecule in addition to perfect shapes of two full-length light chains and two full-length heavy chains. The functional fragments of the antibody molecule refer to fragments having antigen-binding functions, and include Fab, F(ab'), F(ab')2, Fv, etc.

### Effect of the invention

According to the present disclosure, a marker for prognosis of liver cancer, a composition for estimating the prognosis of liver cancer comprising a substance for detecting change in the expression level of the marker for prognosis of liver cancer, a kit for estimating the prognosis of liver cancer comprising the composition for estimating the prognosis of liver cancer, a method for estimating the prognosis of liver cancer using the marker for prognosis of liver cancer, and a method for screening a therapeutic agent for liver cancer using the marker for prognosis of liver cancer.

The marker for prognosis of liver cancer can be usefully used for simple and correct prognosis estimation in patients of liver cancer. Further, the physiological functions of the marker may be directly involved in occurrence or development of liver cancer. Accordingly, the marker can be usefully used for studying mechanism of occurrence or development of liver cancer or as a target for developing a therapeutic agent for liver cancer.

The above markers for prognosis of liver cancers are those that are more significant, highly clinically useful, and capable of more correctly predicting the estimation for prognosis of liver cancer, because they were discovered by statistically analyzing tissues of liver cancer obtained from an unprecedented plenty of patients.

### Brief description of drawings

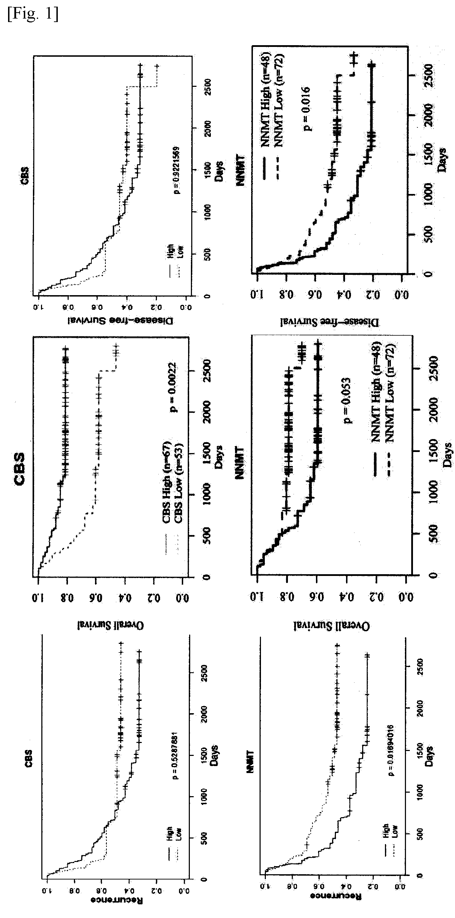

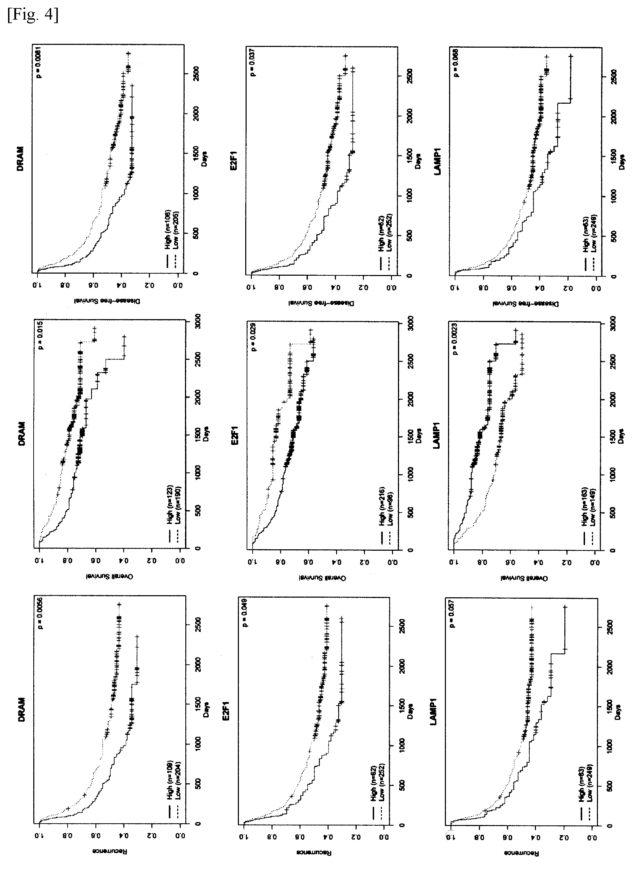

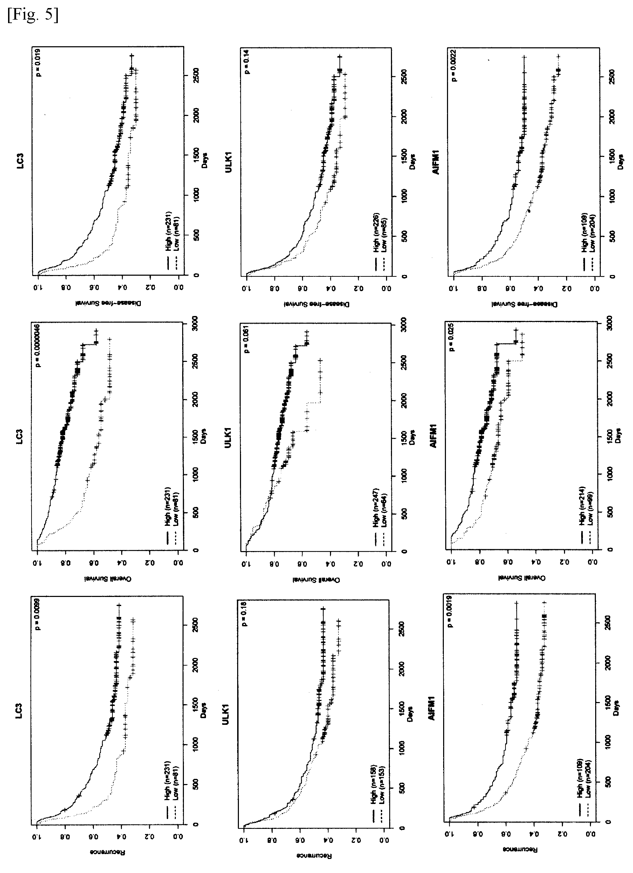

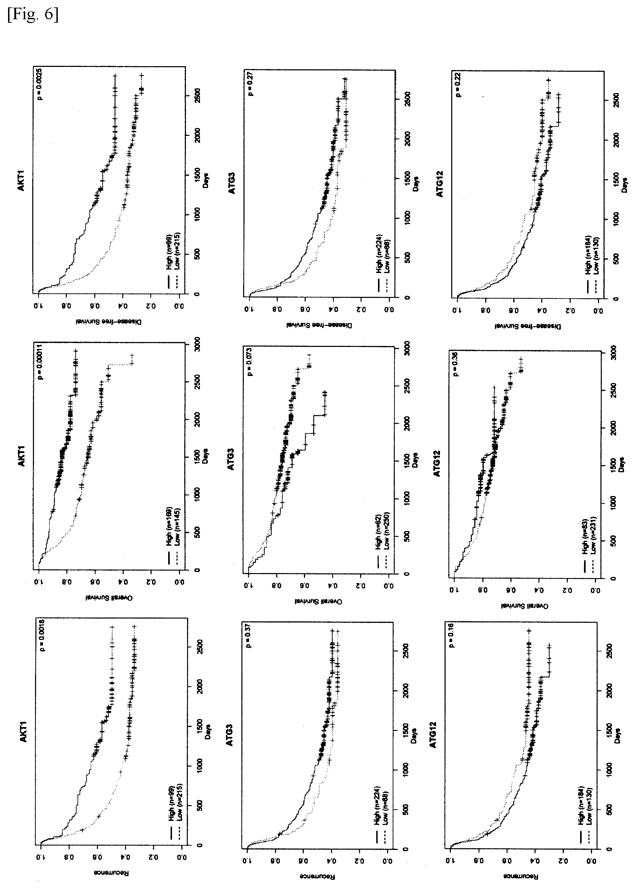

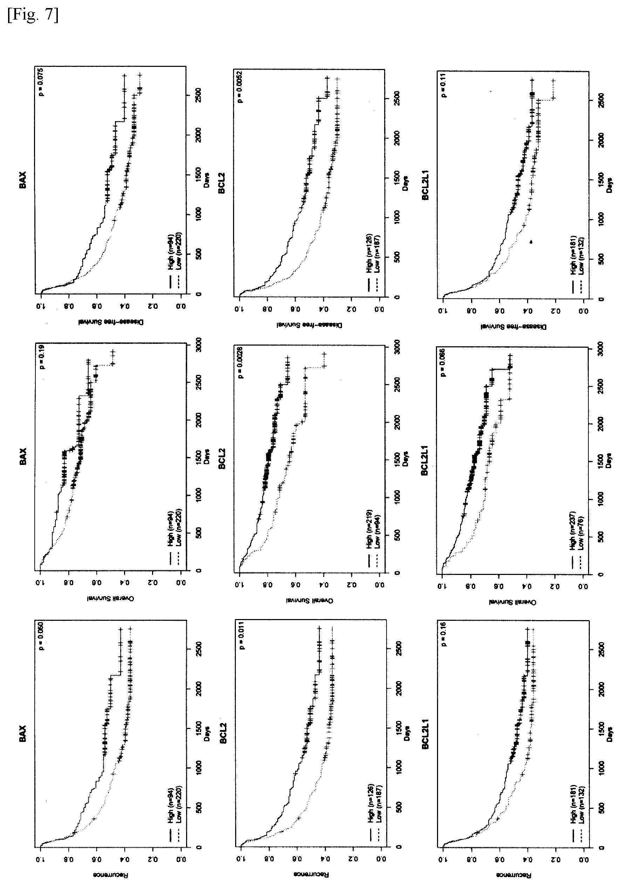

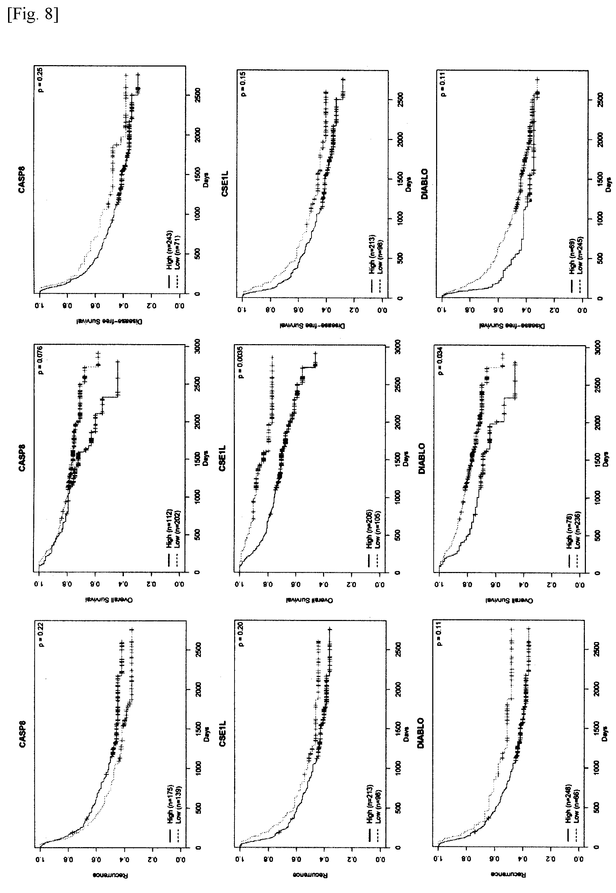

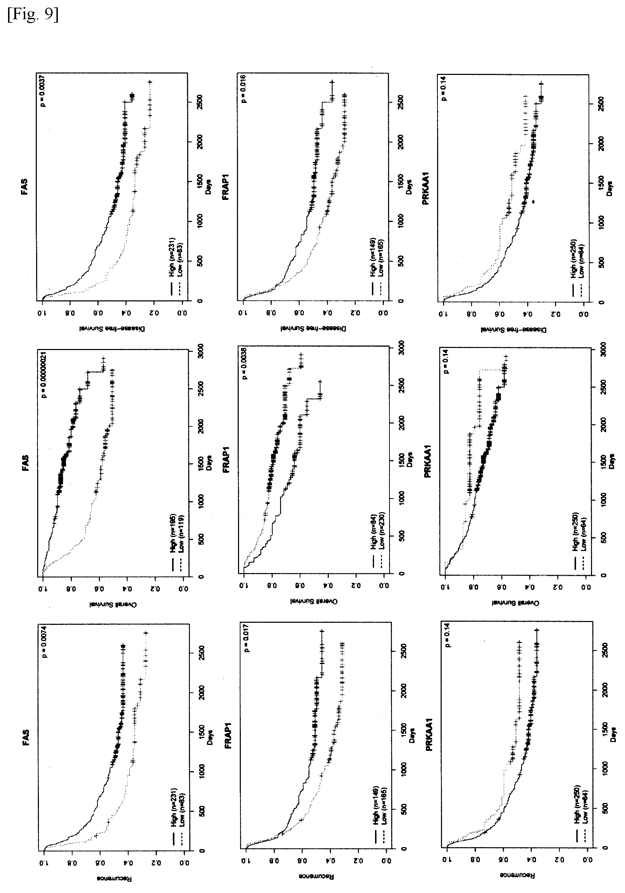

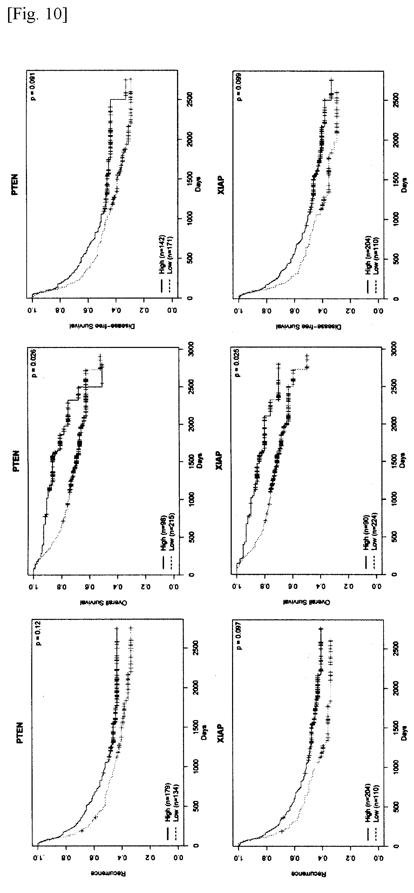

Figs. 1-10 are Kaplan-Meier curves illustrated by measuring the markers for prognosis of liver cancer of the present disclosure in the aspects of recurrence, overall survival, and disease-free survival.

### Best embodiments for carrying out the invention

Hereinafter, the present invention will be explained in detail in examples 1-3; the other examples are comparative examples.

The examples are described only to help understand the present invention, but not to limit the scope of the present invention in any way.

### Embodiments for carrying out the invention

### Example 1: RNA extraction and cDNA synthesis

Liver cancer tissue and adjacent normal tissue harvested from 120 patients of liver cancer whose liver cancer occurrence was diagnosed and its development was confirmed were obtained. RNA of each of the tissues was extracted and cDNA was synthesized according to the following methods.

Total RNA was extracted from liver cancer tissue and adjacent normal tissue using RNeasy Minikit (Qiagen, Germany) according to the manufacturer's instructions. The total RNA of the obtained RNA extract was weighed using Bioanalyzer 2100 (Agilent Technologies, USA). DNase I treatment was performed in the extraction step to remove contaminated genomic DNA from the RNA extract. The sample containing 4 µg of total RNA was incubated with 2 µl of 1 µM oligo d(T)18 primer (Genotech, Korea) at 70□ for 7 minutes and cooled down on ice for 5 minutes. An enzyme mix was separately prepared [in a total volume of 11 µl by adding 2 µl of 0.1 M DTT (Duchefa, Netherlands), 2 µl of 10× reverse-transcription buffer, 5 µl of 2 mM dNTP, 1 µl of 200 U/µl MMLV reverse-transcriptase, and 1 µl of 40 U/µl RNase inhibitor (Enzynomics, Korea)]. After adding the enzyme mix to the samples containing the RNA, they were incubated for 90 minutes at 42°C, and then were incubated at 80°C for 10 minutes to inactivate reverse-transcriptase. The above samples were brought up to a final volume of 400 µl by adding diethylpyrocarbonate (DEPC)-treated water.

### Example 2: Quantitative real-time PCR

Real-time PCR amplifications were carried out for each of the cDNA samples obtained from Example 1, using PRISM 7900HT (Applied Biosystems, USA) according to the manufacturer's instructions, on the following two genetic markers:
CBS and
NNMT.

The real-time PCR analysis was performed in a total volume of 10 µl inclduing 5 µl of 2× Taqman gene expression master mix (Applied Biosystems, USA), 1 µl of each of 5 µM forward and reverse primers, 1 µl of 1 µM probe (Genotech, Korea), and 2 µl of cDNA (in the case of a control group, the same amount of water). The amplifications were performed with a cycle of a step of dissociation at 95°C for 10 minutes, followed by a step of dissociation at 95°C for 15 seconds; and a step of synthesis at 60°C for 1 minute. The primer and probe sequences were designed using Primer Express 3.0 (Applied Biosystems, USA) and all the probe sequences were labelled with FAM at the 5' end and with TAMRA at the 3' end. The following primer and probe sequences in the below Table 1 were used for each of the markers:

**Table 1**

| **Market** | **Sequence (SEQ ID NO)** |
|---|---|
| CABS | |
| F | GTTGGCAAAGTCATCTACAAGCA (1) |
| R | GGGCGAAGTGGTCCATCTC (2) |
| P | ACGCTGGGCAGGCTCTCGCAC (3) |

| NNMT | |
|---|---|
| F | TTGAGGTGATCTCGCAAAGTTATT (4) |
| R | CTCGCCACCAGGGAGAAA (5) |
| P | CCACCATGGCCAACAACGAAGGAC (6) |

| | |
|---|---|
| F: forward primer R: reverse primer P: probe | |

Expression of each of the marker genes was measured in triplicate, and then standardized by subtracting the average expression of 5 types of reference genes (B2M, GAPDH, HMBS, HPRT1, and SDHA). CT (the number of cycles required to achieve a threshold) of each of the markers was measured, and the ΔCT value (CT of each of the markers minus average CT of the reference genes) was calculated. The mRNA copy number was calculated as 2^{-ΔPT}. Standard curves were constructed from the results of simultaneous amplifications of serial dilutions of the cDNA samples.

### Example 3: Statistical analysis

In consideration of the standardized expression of each of the markers obtained from Example 2, and the progress of the patients who have provided liver cancer tissues, Kaplan-Meier curves were completed, and then significance analysis was performed.

Based on the progress of 120 patients who have provided liver cancer tissues, for the respective cases of recurrence, overall survival, and disease-free survival, the patients were listed in the ascending order of the period. The interval survival rate (or interval recurrence rate) was calculated by dividing the number of survivors (or patients with recurrence) by the number of patients exposed to a risk. The cumulative survival rate (or cumulative recurrence rate) is conditional probability, which was calculated by multiplying the previous cumulative survival rate (or cumulative recurrence rate) by the current interval survival rate (or interval recurrence rate). Kaplan-Meier curves were constructed as step functions with the horizontal axis of survival time (or observation period) and the vertical axis of cumulative survival rate (or cumulative reoccurrence rate).

For each of the markers, Kaplan-Meier curves with regard to recurrence, overall survival, and disease-free survival were completed. The expression of each of the markers that was measured in Example 2 was classified into high expression and low expression based on statistically significant references that were determined experimentally, and the cases of high expression and low expression for each of the markers were separated from each other to complete Kaplan-Meier curves. The completed Kaplan-Meier curves are illustrated in Fig. 1.

As can be confirmed from Fig. 1, in the Kaplan-Meier curves completed with respect to recurrence, overall survival, and disease-free survival, each of the markers forms curves where cases of high expression and low expression are distinctively distinguished from each other. This means that there are remarkable differences in interval recurrence rate or interval survival rate, and cumulative recurrence rate or cumulative survival rate based thereon between the cases where each of the markers shows high expression and low expression, and that consequently, the expression patterns of each of the markers can be an index showing recurrence possibility or survival possibility of patients.

Significance tests were performed by log-rank test with respect to each of the markers and their combination by calculating observation values and expected values at every point of recurrence or death to obtain Chi-square test statistics. Thereby, p-values were calculated, and the calculated p-values are as shown in the below Table 2.

**Table 2**

| | **P-value** | | |
|---|---|---|---|
| **Market** | **Recurrence** | **Overall survival** | **Disease-free survival** |
| CBS | 0.52879 | 0.00221 | 0.92216 |
| NNMT | 0.01694 | 0.05333 | 0.01649 |
| CBS_NNMT | 0.03441 | 0.03916 | 0.03641 |

As can be confirmed from the above Table 2, each of the markers or their combination shows p-values low enough to be considered significant in terms of all of recurrence, overall survival, and disease-free survival. In particular, in the case of the combination of the two markers, all the p-values for recurrence, overall survival, and disease-free survival were less than 0.05, which is desirable. As a p-value becomes lower, the statistical significance becomes higher. Thus, the low p-values suggest that the estimation for prognosis of liver cancer by each of the markers or their combination is highly accurate.

### Example 4: Discovery of an additional marker

Except for experimenting with liver cancer tissue and adjacent normal tissue obtained from 185 patients of liver cancer, an experiment was performed in the same manner as in Examples 1 to 3, and thereby Kaplan-Meier curves and p-values were obtained by using the following gene as a marker:
TKT.

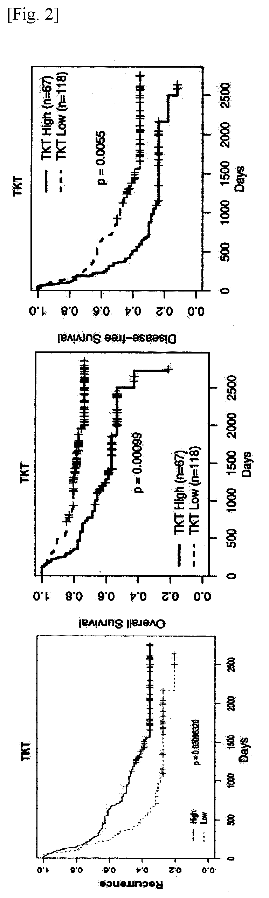

The used primers and probe are as shown in the following Table 3; Kaplan-Meier curves are as shown in Fig. 2; and the calculated p-values are as shown in the following Table 4.

**Table 3**

| **Market** | **Sequence (SEQ ID NO)** |
|---|---|
| TKT | |
| F | GAGGCTGTGTCCAGTGCAGTAG (7) |
| R | CCACTTCTTGGTACCCGGTTAA (8) |
| P | CCTGGCATCACTGTCACCCACCTG (9) |

| | |
|---|---|
| F: forward primer R: reverse primer P: probe | |

**Table 4**

| | **P-value** | | |
|---|---|---|---|
| **Marker** | **Recurrence** | **Overall survival** | **Disease-free survival** |
| TKT | 0.03096 | 0.00099 | 0.00546 |

As can be seen from Fig. 2, in Kaplan-Meier curves completed with respect to recurrence, overall survival, and disease-free survival, the above marker forms curves where cases of high expression and low expression are distinctively distinguished from each other. This means that there are remarkable differences in interval recurrence rate or interval survival rate and cumulative recurrence rate or cumulative survival rate based thereon between the cases where the marker is in high expression and low expression, and that consequently, the expression pattern of the marker can be an index showing recurrence possibility or survival possibility of patients.

As can be confirmed from the above Table 4, the above marker shows a p-value of less than 0.05 with respect to all of recurrence, overall survival, and disease-free survival, which is desirably low. As a p-value becomes lower, the statistical significance becomes higher. Thus, the low p-value suggests that the estimation for prognosis of liver cancer by the marker is highly accurate.

### Example 5: Discovery of an additional marker

Except for experimenting with liver cancer tissue and adjacent normal tissue obtained from 369 patients of liver cancer, an experiment was performed in the same manners as in Examples 1 to 3, and thereby Kaplan-Meier curves and p-values were obtained by using the following 23 genes as markers:
AIFM1
AKT1
ATG3
ATG5
ATG7
ATG12
BAX
BCL2
BCL2L1
BNIP3
CASP8
CSE1L
DIABLO
DRAM
E2F1
FAS
FRAP1
LAMP1
LC3[MAP1LC3A]
PRKAA1
PTEN
ULK1 and
XIAP

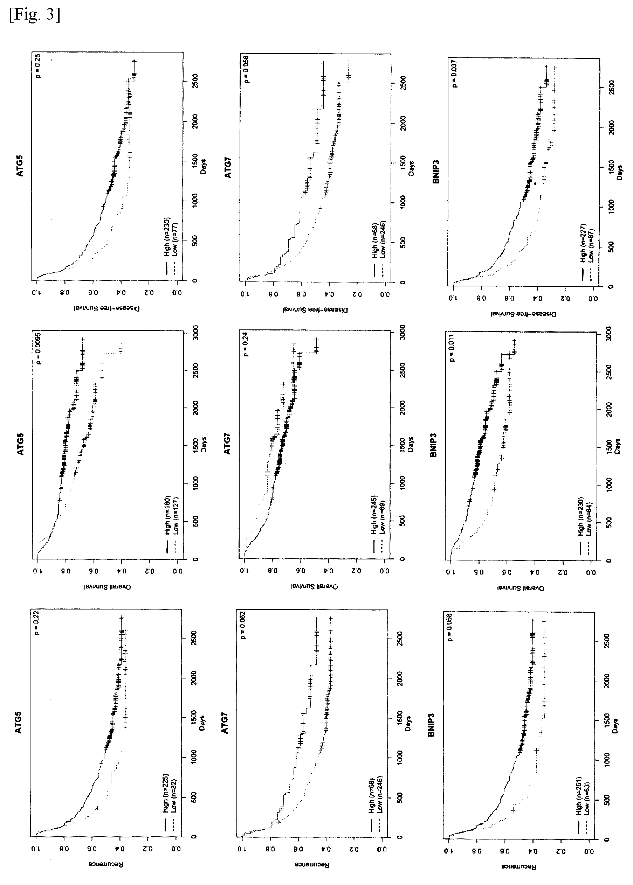

The used primers and probes are as shown in the following Table 5; Kaplan-Meier curves are as shown in Figs. 3 to 10; and the calculated p-values for each of the markers are as shown in the following Table 6. The calculated p-values for combinations of any two types of makers are as shown in the following Tables 7 to 9.

**Table 5**

| Marker | Sequence (SEQ ID NO) | Marker | Sequence (SEQ ID NO] |
|---|---|---|---|
| BCL2 | | PRKAA1 | |
| F | CATGTGTGTGGAGAGCGTCAA (10) | F | GGCAGTTGCCTACCATCTCATAA (46) |
| R | GCCGGTTGAGGTACTCAGTCA (11) | R | GCCGAGTCAGGTGATGATCA (47) |
| P | CCTGGTGGACAACATCGCCCTGT (12) | P | TCTATTTGGCGACAAGCCCACCTGATT (48) |
| FAS | | ATG3 | |
| F | AATGGTGTCAATGAASCCAAAAT (13) | F | CCATTGAAAATCACCCTCATCTG (49) |
| R | GTCATACGCTTCTTTCTTTCCATGA (14) | | CACCTCAGCATGCCTGCAT (50) |
| P | TGACAATGTGCAAGACACAGCAGAACAGAA (15) | R P | CACCACCTCCCATGTGTTCAGTTCACC (51) |
| BAX | | ATG12 | |
| F | GGTTGTCGCCCTTTTCTACTTTG (16) | F | CGACAGCTTCGATTTGAATGAC (52) |
| R | CAGTTCCGCACCTTGGT (17) | R | GGAAGCGTCGCAAAGGA (53) |
| P | CAGCAAACTGGTGCTCAAGGOC CT (18) | P | TGTAATTGCGTGCCCGTACTCCGGC (54) |
| BCL2L1 | | ATG5 | |
| F | GATTGCCTTTGTTTTGATGTTTGT (19) | F | TTTCCTCCACTGCCATCATTAA (55) |
| R | GGAAAGGiQAACCCAGGTTAGTG (20) | | GGCCAAAGGTTTCAGCTTCA (66) |
| P | CAGAATTGATCATDTCCCCCCACTCTCC (21) | R P | CCTCAGCTGTGACATGAAAGACTTACCGG (67) |
| AIFM1 | | ATG7 | |
| F | TGAAGATCTCAATGAAGTAGCCAAACTAT (22) | F | CGGATGAATGAGCCTCCAA (58) |
| R | CTGCAGTGGGTTTGCCAAT (23) | R | GGACATTATCAAAGCGTGAAAGAA (59) |
| P | CAACATTCATGAAGACTGAAGCCCCACA (24) | P | TCTTGGGCTTGTGCCTGAGCAGATC (60) |
| CASP8 | | BNIP3 | |
| F | CCTTCTGATTGATGGTGCTATTTTG (25) | F | TTCCCCCCAAGGAGTTCCT (61) |
| R | GCGGTGAGCCGAGATCAC (26) | R | CGCTCGTGTTCCTCATGCT (62) |
| P | CAGAATCTCGCTCTGTCGCCCAGG (27) | P | ACCCGAAGCGCACGGCCAC (63) |
| CSE1L | | DRAM | |
| F | GCATGATCCTGTAGGTCAAATGG (28) | F | CAGCCGCCTTCATTATGTCGTA (64) |
| R | CAGGCGGTAGACAACTTGTGAA (29) | R | TGGAGGTGTTGTTCCCGTATC (65) |
| P | AATAACCCCAAAATTCACCTGGCACAGTCA (30) | P | TGCTCTCCGGCCACSTCAACC (66) |
| XIAP | | LAMP1 | |
| F | GACAGGCCATCTGAGACACATG (31) | F | TGCACTCAGATTTAAGCCTTACAAA (67) |
| R | AGCATAGTCTGGCCAGTTCTGAA (32) | R | TCACCACGAGTGACCTTCATG (66) |
| P | AGACACCATATACCCGACGAACCCTGCC (33) | P | AAGCCTCTGGCCGTCACACGTAGG (69) |
| DIABLO | | LC3 | |
| F | AATCACATTCAGCTGGTGAAACTG (34) | F | AACCAGCACAGCATGGTGAGT (70) |
| R | TGCCAGCTTGGTTTCTGCTT (35) | R | CCTCGTCTTTCTCOTCCTCGTA (71) |
| P | AAGAGGTGCACCAGCTCTCCCGG (36) | P | TCCACGCCCATCGCGACA (72) |
| AKT1 | | ULK1 | |
| F | TCTCGGGTGCATTTGAGAGAA (37) | F | TCGCGGCCGCATGT (73) |
| R | ACAGCACAAAAACGTCTTTCCA (38) | R | AAGGGTCCAGCACTATCAAGAGA (74) |
| P | CCACGCTGTCCTCTCGAGCGCA (39) | P | AGCAGGTCCTGCGCGCCTCAAC (75) |
| PTEN | | E2F1 | |
| F | TGGCGGAACTTGCAATCC (40) | F | CTGGACCACCTGATGAATATCTGT (76) |
| R | GCTGAGGGAACTCAAAGTACATGA (41) | R | CAATGCTACGAAGGTCCTGACA (77) |
| P | ATATTCCTCCAATTCAGGACCCACACGAC (42) | P | CAGCTGCGCCTGCTCTCGGA (78) |
| FRAP1 | | | |
| F | AGGCCGCATTGTCTCTATCAA (43) | | F: forward primer |
| R | GCAGTAAATGCAGGTAGTCATGCA (44) | | R: reverse primer |
| P | TGCAATCCAGCTGTTTGGCGCC (45) | | P: probe |

**Table 6**

| | **P-value** | | |
|---|---|---|---|
| **Marker** | **Recurrence** | **Overall survival** | **Disease-free survival** |
| BNIP3 | 0.02022 | 0.00354 | 0.00627 |
| DRAM | 0.00669 | 0.00512 | 0.00953 |
| LAMP1 | 0.02721 | 0.00225 | 0.02398 |
| LC3 | 0.01134 | 0.00003 | 0.02196 |
| AIFM1 | 0.00117 | 0.04046 | 0.00114 |
| AKT1 | 0.00042 | 0.00003 | 0.00093 |
| BCL2 | 0.02467 | 0.00658 | 0.01477 |
| BCL2L1 | 0.04975 | 0.01110 | 0.04464 |
| FAS | 0.00701 | 0.00000 | 0.00255 |
| FRAP1 | 0.02911 | 0.00421 | 0.01940 |
| BAX | 0.02799 | 0.10527 | 0.03650 |
| ULK1 | 0.05250 | 0.04146 | 0.04745 |
| PTEN | 0.09680 | 0.00893 | 0.07908 |
| CSE1L | 0.08817 | 0.00134 | 0.05249 |
| XIAP | 0.11658 | 0.03111 | 0.15221 |
| ATG5 | 0.12114 | 0.00880 | 0.14045 |
| E2F1 | 0.22181 | 0.04046 | 0.14706 |
| DIABLO | 0.19717 | 0.04542 | 0.24942 |
| ATG3 | 0.28588 | 0.39987 | 0.17641 |
| ATG7 | 0.06493 | 0.22432 | 0.06122 |
| ATG12 | 0.19654 | 0.13755 | 0.26693 |
| CASP8 | 0.10122 | 0.15557 | 0.18655 |
| PRKAA1 | 0.18647 | 0.19015 | 0.32981 |

**Table 7**

| | **P-value** | | | | **P-value** | | |
|---|---|---|---|---|---|---|---|
| **Marker** | Recurrence | Overall survival | Disease-free survival | **Marker** | Recurrence | Overall survival | Disease-free survival |
| ATG5_ATG7 | 0.15663 | 0.00589 | 0.19962 | BNIP3_DRAM | 0.02162 | 0.00173 | 0.10550 |
| ATG5_BNIP3 | 0.04903 | 0.00536 | 0.04662 | BNIP3_E2F1 | 0.09158 | 0.00221 | 0.02682 |
| ATG5_DRAM | 0.00308 | 0.02402 | 0.00859 | BNIP3_LAMP1 | 0.03650 | 0.00947 | 0.01819 |
| ATG5_E2F1 | 0.02165 | 0.00258 | 0.02076 | BNIP3_LC3 | 0.00130 | 0.00004 | 0.00260 |
| ATG5_LAMP1 | 0.01654 | 0.00270 | 0.02686 | BNIP3_ULK1 | 0.10639 | 0.01120 | 0.06007 |
| ATG5_LC3 | 0.00269 | 0.00001 | 0.00045 | BNIP3_AIFM1 | 0.00084 | 0.00477 | 0.00111 |
| ATG5_ULK1 | 0.08370 | 0.02165 | 0.02992 | BNIP3_AKT1 | 0.00036 | 0.00001 | 0.00067 |
| ATG5_AIFM1 | 0.00073 | 0.00539 | 0.00031 | BNIP3_ATG3 | 0.02013 | 0.00371 | 0.00590 |
| ATG5_AKT1 | 0.00013 | 0.00075 | 0.00025 | BNIP3_ATG12 | 0.02547 | 0.00127 | 0.00207 |
| ATG5_ATG3 | 0.16614 | 0.01176 | 0.14048 | BNIP3_BAX | 0.05882 | 0.00105 | 0.04363 |
| ATG5_ATG12 | 0.14245 | 0.02291 | 0.17473 | BNIP3_BCL2 | 0.00632 | 0.00002 | 0.00088 |
| ATG5_BAX | 0.06603 | 0.01020 | 0.07166 | BNIP3_BCL2L1 | 0.04550 | 0.00009 | 0.00475 |
| ATG5_BCL2 | 0.01846 | 0.00479 | 0.00904 | BNIP3_CASP8 | 0.09283 | 0.00140 | 0.04177 |
| ATG5_BCL2L1 | 0.01850 | 0.00474 | 0.01292 | BNIP3_CSE1L | 0.05584 | 0.00007 | 0.02126 |
| ATG5_CASP8 | 0.09018 | 0.02486 | 0.15403 | BMIP3_DIA6LO | 0.14797 | 0.00343 | 0.06290 |
| ATG5_CSE1L | 0.20921 | 0.00061 | 0.11020 | BNIP3_FAS | 0.00484 | 0.00000 | 0.00171 |
| ATG5_DIABLO | 0.10373 | 0.00880 | 0.09206 | BNIP3_FRAP1 | 0.01789 | 0.00342 | 0.00838 |
| ATG5_FAS | 0.00134 | 0.00000 | 0.00066 | BNIP3_PRKAA1 | 0.10235 | 0.00096 | 0.00627 |
| ATG5_FRAP1 | 0.01550 | 0.00835 | 0.01724 | BNIP3_PTEN | 0.01871 | 0.00504 | 0.00670 |
| ATG5_PRKAA1 | 0.15990 | 0.02758 | 0.17648 | BNIP3_XIAP | 0.07272 | 0.00671 | 0.02349 |
| ATG5_PTEN | 0.10454 | 0.02060 | 0.12330 | DRAM_E2F1 | 0.05933 | 0.02423 | 0.03906 |
| ATG5_XIAP | 0.07805 | 0.00032 | 0.07617 | DRAM_LAMP1 | 0.00436 | 0.00232 | 0.01206 |
| ATG7_BNIP3 | 0.03152 | 0.00141 | 0.00392 | DRAM_LC3 | 0.00890 | 0.00003 | 0.02502 |
| ATG7_DRAM | 0.00669 | 0.01265 | 0.01042 | DRAM_ULK1 | 0.02697 | 0.00201 | 0.02857 |
| ATG7_E2F1 | 0.19080 | 0.05246 | 0.11006 | DRAM_AIFM1 | 0.01153 | 0.02446 | 0.03577 |
| ATG7_LAMP1 | 0.04615 | 0.00468 | 0.04289 | DRAM_AKT1 | 0.00001 | 0.00000 | 0.00002 |
| ATG7_LC3 | 0.01550 | 0.00017 | 0.04352 | DRAM_ATG3 | 0.03660 | 0.04492 | 0.00895 |
| ATG7_ULK1 | 0.02772 | 0.01613 | 0.01082 | DRAM_ATG12 | 0.02283 | 0.00308 | 0.01500 |
| ATG7_AIFM1 | 0.00031 | 0.05341 | 0.00075 | DRAM_BAX | 0.00026 | 0.00145 | 0.00259 |
| ATG7_AKT1 | 0.00065 | 0.00003 | 0.00100 | DRAM_BCL2 | 0.00427 | 0.00133 | 0.00233 |
| ATG7_ATG3 | 0.25700 | 0.08356 | 0.30334 | DRAM_BCL2L1 | 0.00017 | 0.00008 | 0.00157 |
| ATG7_ATG12 | 0.10515 | 0.08168 | 0.26653 | DRAM_CASP8 | 0.00455 | 0.00212 | 0.00859 |
| ATG7_BAX | 0.05367 | 0.03772 | 0.06089 | DRAM_CSE1L | 0.06106 | 0.01159 | 0.09206 |
| ATG7_BCL2 | 0.02532 | 0.00107 | 0.01848 | DRAM_DIABLO | 0.03026 | 0.01389 | 0.04108 |
| ATG7_BCL2L1 | 0.04597 | 0.01275 | 0.03730 | DRAM_FAS | 0.00065 | 0.00000 | 0.00040 |
| ATG7_CASP8 | 0.28393 | 0.03978 | 0.12008 | DRAM_FRAP1 | 0.00109 | 0.01182 | 0.00172 |
| ATG7_CSE1L | 0.08763 | 0.01256 | 0.04956 | DRAM_PRKAA1 | 0.00917 | 0.01627 | 0.03695 |
| ATG7_DIABLO | 0.14685 | 0.26001 | 0.17462 | DRAM_PTEN | 0.00755 | 0.00455 | 0.02852 |
| ATG7_FAS | 0.00569 | 0.00000 | 0.00306 | DRAM_XIAP | 0.01616 | 0.00099 | 0.01646 |
| ATG7_FRAP1 | 0.03716 | 0.17212 | 0.03870 | E2F1_LAMP1 | 0.00316 | 0.00091 | 0.00459 |
| ATG7_PRKAA1 | 0.22418 | 0.15039 | 0.06122 | E2F1_LC3 | 0.01172 | 0.00012 | 0.03729 |
| ATG7_PTEN | 0.22594 | 0.00809 | 0.31309 | E2F1_ULK1 | 0.03342 | 0.00245 | 0.02811 |
| ATG7_XIAP | 0.09661 | 0.04494 | 0.13129 | E2F1_AIFM1 | 0.00133 | 0.00991 | 0.00212 |

**Table 8**

| | **P-value** | | | | **P-value** | | |
|---|---|---|---|---|---|---|---|
| **Marker** | Recurrence | Overall survival | Disease-free survival | **Marker** | Recurrence | Overall survival | Disease-free survival |
| E2F1_AKT1 | 0.00031 | 0.00001 | 0.00035 | LC3_FRAP1 | 0.00151 | 0.00002 | 0.00457 |
| E2F1_ATG3 | 0.10870 | 0.04951 | 0.13822 | LC3_PRKAA1 | 0.00463 | 0.00004 | 0.02196 |
| E2F1_ATG12 | 0.16431 | 0.00762 | 0.05090 | LC3_PTEN | 0.00574 | 0.00006 | 0.00676 |
| E2F1_BAX | 0.02714 | 0.01489 | 0.03789 | LC3_XIAP | 0.00112 | 0.00000 | 0.00264 |
| E2F1_BCL2 | 0.00716 | 0.00017 | 0.00361 | ULK1_AIFM1 | 0.00004 | 0.00271 | 0.00000 |
| E2F1_BCL2L1 | 0.03851 | 0.00511 | 0.01052 | ULK1_AKT1 | 0.00009 | 0.00011 | 0.00017 |
| E2F1_CASP8 | 0.07422 | 0.00993 | 0.09363 | ULK1_ATG3 | 0.08868 | 0.02385 | 0.04993 |
| E2F1_CSE1L | 0.02619 | 0.02525 | 0.02747 | ULK1_ATG12 | 0.03242 | 0.01312 | 0.02518 |
| E2F1_DIABLO | 0.14033 | 0.02897 | 0.29365 | ULK1_BAX | 0.10828 | 0.03532 | 0.07295 |
| E2F1_FAS | 0.00084 | 0.00000 | 0.00027 | ULK1_BCL2 | 0.01626 | 0.00077 | 0.01425 |
| E2F1_FRAP1 | 0.02063 | 0.00792 | 0.01632 | ULK1_BCL2L1 | 0.00284 | 0.00186 | 0.00166 |
| E2F1_PRKAA1 | 0.02286 | 0.03169 | 0.14706 | ULK1_CASP8 | 0.04841 | 0.02399 | 0.03932 |
| E2F1_PTEN | 0.15150 | 0.01199 | 0.07354 | ULK1_CSE1L | 0.01897 | 0.01106 | 0.01575 |
| E2F1_XIAP | 0.05458 | 0.00110 | 0.02301 | ULK1_DIABLO | 0.03413 | 0.03998 | 0.04386 |
| LAMP1_LC3 | 0.00212 | 0.00007 | 0.00381 | ULK1_FAS | 0.00271 | 0.00000 | 0.00185 |
| LAMP1_ULK1 | 0.00594 | 0.01664 | 0.00165 | ULK1_FRAP1 | 0.00118 | 0.04037 | 0.00059 |
| LAMP1_AIFM1 | 0.00488 | 0.00132 | 0.00362 | ULK1_PRKAA1 | 0.02697 | 0.02975 | 0.02504 |
| LAMP1_AKT1 | 0.00078 | 0.00010 | 0.00176 | ULK1_PTEN | 0.06942 | 0.05032 | 0.04631 |
| LAMP1_ATG3 | 0.00940 | 0.00019 | 0.00482 | ULK1_XIAP | 0.00151 | 0.00139 | 0.00037 |
| LAMP1_ATG12 | 0.02021 | 0.00089 | 0.02300 | AIFM1_AKT1 | 0.00002 | 0.00053 | 0.00002 |
| LAMP1_BAX | 0.01005 | 0.00146 | 0.00913 | AIFM1_ATG3 | 0.00113 | 0.01802 | 0.00069 |
| LAMP1_BCL2 | 0.01957 | 0.00454 | 0.01225 | AIFM1_ATG12 | 0.00255 | 0.00589 | 0.00071 |
| LAMP1_BCL2L1 | 0.00162 | 0.00109 | 0,00204 | AIFM1_BAX | 0.00088 | 0.00603 | 0.00112 |
| LAMP1_CASP8 | 0.01155 | 0.00654 | 0.01492 | AIFM1_BCL2 | 0.00951 | 0.00065 | 0.00520 |
| LAMP1_CSE1L | 0.00260 | 0.00167 | 0.00072 | AIFM1_BCL2L1 | 0.00031 | 0.00046 | 0.00041 |
| LAMP1_DIABLO | 0.00904 | 0.00243 | 0.00405 | AIFM1_CASP8 | 0.00097 | 0.00310 | 0.00179 |
| LAMP1_FAS | 0.00039 | 0.00000 | 0.00028 | AIFM1_CSE1L | 0.00475 | 0.01283 | 0.00686 |
| LAMP1_FRAP1 | 0.01773 | 0.00289 | 0.01815 | AIFM1_DIABLO | 0.00186 | 0.04046 | 0.00307 |
| LAMP1_PRKAA1 | 0.01790 | 0.00020 | 0.02398 | AIFM1_FAS | 0.01967 | 0.00000 | 0.01279 |
| LAMP1_PTEN | 0.02907 | 0.00122 | 0.01067 | AIFM1_FRAP1 | 0.00010 | 0.03628 | 0.00013 |
| LAMP1_XIAP | 0.01038 | 0.00164 | 0.01272 | AIFM1_PRKAA1 | 0.00090 | 0.02749 | 0.00114 |
| LC3_ULK1 | 0.00136 | 0.00002 | 0.00296 | AIFM1_PTEN | 0.00101 | 0.00867 | 0.00123 |
| LC3_AIFM1 | 0.00003 | 0.00015 | 0.00063 | AIFM1_X1AP | 0.00438 | 0.00388 | 0.00571 |
| LC3_AKT1 | 0.00013 | 0.00001 | 0.00006 | AKT1_ATG3 | 0.00037 | 0.00003 | 0.00043 |
| LC3_ATG3 | 0.00560 | 0.00002 | 0.00871 | AKT1_ATG12 | 0.00043 | 0.00037 | 0.00072 |
| LC3_ATG12 | 0.00646 | 0.00003 | 0.01685 | AKT1_BAX | 0.00067 | 0.00074 | 0.00061 |
| LC3_BAX | 0.00250 | 0.00019 | 0.00638 | AKT1_BCL2 | 0.00027 | 0.00001 | 0.00018 |
| LC3_BCL2 | 0.00332 | 0.00078 | 0.00666 | AKT1_BCL2L1 | 0.00310 | 0.00025 | 0.00265 |
| LC3_BCL2L1 | 0.00485 | 0**.**00009 | 0.01206 | AKT1_CASP8 | 0**.**00056 | 0.00005 | 0.00050 |
| LC3_CASP8 | 0.02236 | 0.00005 | 0.04875 | AKT1_CSE1L | 0.00014 | 0.00000 | 0.00024 |
| - LC3_CSEIL | 0.03137 | 0**.**00017 | 0.04571 | AKT1_DIABLO | 0.00059 | 0.00004 | 0.00100 |
| LC3_DIABLO | 0.01354 | 0.00003 | 0.01930 | AKT1_FAS | 0.00016 | 0.00000 | 0.00012 |
| LC3_FAS | 0.00184 | 0.00000 | 0.00340 | AKT1_FRAP1 | 0.00068 | 0.00005 | 0.00032 |

**Table 9**

| | **P-value** | | | | **P-value** | | |
|---|---|---|---|---|---|---|---|
| **Marker** | Recurrence | Overall | Disease-free survival" | **Marker** | Recurrence | Overall survival | Disease-free survival |
| AKT1_PRKAA1 | 0.00068 | 0.00001 | 0.00066 | BCL2_PTEN | 0.02127 | 0.00302 | 0.00809 |
| AKT1_PTEN | 0.00071 | 0.00018 | 0.00059 | BCLS₂_XIAP | 0.02736 | 0,00290 | 0.01938 |
| AKT1_XIAP | 0.00116 | 0.00118 | 0.00100 | BCL2L_CASP8 | 0.08272 | 0.01882 | 0.05650 |
| ATG3_A7G12 | 0.15445 | 0.14873 | 0.25699 | BCL2L1_CSE1L | 0.01769 | 0.00000 | 0.00700 |
| ATG3_BAX | 0.02736 | 0.04408 | 0.02059 | BCL2L1_DIABLO | 0.01088 | 0.01110 | 0.02757 |
| ATG3_BCL2 | 0.01955 | 0.01158 | 0.01094 | BCL2L1_FAS | 0.00070 | 0.00000 | 0.00023 |
| ATG3_BCL2L1 | 0.03868 | 0.01520 | 0.01342 | BCL2L1_FRAP1 | 0.01802 | 0.01215 | 0.01427 |
| ATG3_CASP8 | 0.18048 | 0.21489 | 0.10896 | BCL2L1_PRKAA1 | 0.03436 | 0.00833 | 0.04360 |
| ATG3_CSE1L | 0.09776 | 0.01335 | 0.20272 | BCL2L1_PTEN | 0.04370 | 0.00257 | 0.01352 |
| ATG3_DIABLO | 0.18448 | 0.28800 | 0.06279 | BCL2L1_XIAP | 0.02829 | 0.02026 | 0.03847 |
| ATG3_FAS | 0.00701 | 0.00000 | 0.00048 | CASP8_CSE1L | 0.12292 | 0.00721 | 0.05678 |
| ATG3_FRAP1 | 0.03988 | 0.08529 | 0.04513 | CASP8_DIABLO | 0.17491 | 0.18725 | 0.28080 |
| ATG3_PRKAA1 | 0.12105 | 0.24500 | 0.14597 | CASPB_FAS | 0.00256 | 0.00000 | 0.00136 |
| ATG3_PTEN | 0.37245 | 0.01382 | 0.23336 | CASP8_FRAP1 | 0.05703 | 0.18479 | 0.03680 |
| ATG3_XIAP | 0.04243 | 0.06838 | 0.10574 | CASP8_PRKAA1 | 0.12642 | 0.22765 | 0.18655 |
| ATG12_BAX | 0.03524 | 0_02723 | 0.03497 | CASP8_PTEN | 0.19020 | 0.23066 | 0.29905 |
| ATG12_BCL2 | 0.01507 | 0.00202 | 0.01477 | CASP8_XIAP | 0.21861 | 0.01829 | 0.18520 |
| ATG12-BCL2L1 | 0.03069 | 0.01334 | 0.03303 | CSE1L_DIABLO | 0.08685 | 0.00226 | 0.13233 |
| ATG12_CASP8 | 0.03429 | 0.25396 | 0.24041 | CSE1L_FAS | 0.00106 | 0.00000 | 0.00045 |
| ATG12_CSE1L | 0.12720 | 0.00375 | 0.05225 | CSE1L_FRAP1 | 0.02406 | 0.00218 | 0.00208 |
| ATG12_DIABLO | 0.17794 | 0.13391 | 0.16379 | CSE1L_PRKAA1 | 0.11173 | 0.01837 | 0.05249 |
| ATG12_FAS | 0.00629 | 0.00000 | 0.00255 | CSE1L_PTEN | 0.06721 | 0.00371 | 0.05229 |
| ATG12_FRAP1 | 0.01939 | 0.06615 | 0.02144 | CSE1L_XIAP | 4.01878 | 0.00422 | 0.02168 |
| ATG12_PRAA1 | 0.15988 | 0.07166 | 0.25753 | DIABLO_FAS | 0.00946 | 0.00000 | 0.00393 |
| ATG12_PTEN | 0.02937 | 0.07228 | 0.29766 | DIABLO_FRAP1 | 0.00193 | 0.00706 | 0.00190 |
| ATG12_XIAP | 0.1451 | 0.10198 | 0.15802 | DIABLO_PRKAA1 | 0.39049 | 0.22100 | 0.21006 |
| BAX_BCL2 | 0.01443 | 0.00904 | 0.01411 | DIABLO_PTEN | 0.18426 | 0.00785 | 0.14222 |
| BAX_BCL2L1 | 0.14757 | 0.01104 | 0.48992 | DIABLO_XIAP | 0,21769 | 0.03111 | 0.25563 |
| BAX_CASP8 | 0.1873 | 0.34789 | 0.19927 | PAS_FRAP1 | 0.00603 | 0.00000 | 0.00103 |
| BAX_CSE1L | 0.00618 | 0.00328 | 0.00814 | FAS_PRKAA1 | 0.00750 | 0.00000 | 0.00181 |
| BAX_DIABLO | 0.07894 | 0.10527 | 0.06564 | FAS_PTEN | 0.00701 | 0.00000 | 0.00420 |
| BAX_FAS | 0.00177 | 0.00000 | 0.00025 | FAS_XIAP | 0.00366 | 0.00000 | 0.00154 |
| BAX_FRAP1 | 0.01460 | 0.17898 | 0.01397 | FRAP1_RKAA1 | 0.02661 | 0.19329 | 0.01940 |
| BAX_PRKAA1 | 0.05687 | 0.11741 | 0.03650 | FRAP1_PTEN | 0.02321 | 0.05015 | 0.01473 |
| BAX_PTEN | 0.02200 | 0.12485 | 0.04668 | FRAP1_XIAP | 0.03353 | 0.03111 | 0.01147 |
| BAX_XIAP | 0.06035 | 0.02569 | 0.02866 | PRKAA1_PTEN | 0.17904 | 0.00186 | 0.07802 |
| BCL2_BCL2L1 L1 | 0.01560 | 0.00096 | 0.01055 | PRKAA1_XIAP | 0.18116 | 0.02727 | 0.15221 |
| BCL2_CASP8 | 0.03785 | 0.00703 | 0.01883 | PTEN_XIAP | 0.03963 | 0.01364 | 0.17000 |
| BCL2_CSE1L | 0.00982 | 0.00417 | 0.00709 | | | | |
| BCL2_DIABLO | 0.02945 | 0.00215 | 0.00896 | | | | |
| BCL2_FAS | 0.00235 | 0.00000 | 0.00069 | | | | |
| BCL2_FRAP1 | 0.02771 | 0.00437 | 0.01162 | | | | |
| BCL2_PRKAA1 | 0.00963 | 4.00180 | 0.01691 | | | | |

As can be confirmed from Figs. 3 to 10, in Kaplan-Meier curves completed with respect to recurrence, overall survival, and disease-free survival, each of the markers forms curves where cases of high expression and low expression are distinctively distinguished from each other. This means that there are remarkable differences in interval recurrence rate or interval survival rate, and cumulative recurrence rate or cumulative survival rate based thereon between the cases where each of the markers shows high expression and low expression, and that consequently, the expression patterns of each of the markers can be an index showing recurrence possibility or survival possibility of patients.

As can be confirmed from the above Tables 6 to 9, each of the markers or all the combinations of two types of markers show p-values low enough to be considered significant in terms of all of recurrence, overall survival, and disease-free survival. As a p-value becomes lower, the statistical significance becomes higher. Thus, the low p-values suggest that the estimation for prognosis of liver cancer by each of the markers or their combinations is accurate. In particular, most of each of the markers or any combinations of two types of markers show p-values of less than 0.05, which is desirably low.

All the p-values of combinations of three or more types of markers are less than 0.05. Single markers or combinations of two or more markers which show the lowest p-values, and the corresponding p-values are as shown in the below Table 10.

**Table 10**

| [Recurrence] | | |
|---|---|---|
| Number of markers combined | Marker | P.value |
| 1 | AKT1 | 0.00300 |
| 2 | DRAM_AKT1 | 0.00041 |
| 3 | DRAM_ULK1_AKT1 | 0.00010 |
| 4 | DRAM_ULK1_AKT1_CSE1L | 0.00004 |
| 5 | DRAM_ULK1_KT1_ATG12_CSE1L | 0.00002 |
| 6 | DRAM_E2F1_ULK1_AKT1_CSE1L_FRAP1 | 0.00002 |
| 7 | DRAM_E2F1_ULK1_AKT1_ATG12_CSE1L_FRAP1 | 0.00001 |
| 8 | DRAM_E2F1_ULK1_AKT1_ATG3-ATG12_CSE1_FRAP1 | 0,00001 |

| [Overall survival] | | |
|---|---|---|
| Number of markers combined | Marker | P-value |
| 1 | FAS | 0.00000 |
| 2 | DRAM_FAS | 0.00000 |
| 3 | DRAM_ATG3_FAS | 0.00000 |
| 4 | DRAM_AKT1_CSE1L_FAS | 0.00000 |
| 5 | DRAM_AKT1_ATG3_CSE1L_FAS | 0.00000 |
| 6 | ATG7_DRAM_AKT1_ATG3_CSE1L_FAS | 0.00000 |
| 7 | ATG7_DRAM_AKT1_ATG3_DIABLO_FAS_PRKAA1 | 0.00000 |
| 8 | ATG7_DRAM_AKT1_ATG3_CSE1L_FAS_PRKAA1_XIAP | 0.00000 |

| [Disease-free Survival] | | |
|---|---|---|
| Number of markers combined | Marker | P-value |
| 1 | AKT1 | 0.00220 |
| 2 | DRAM_AKT1 | 0.00049 |
| 3 | DRAM_ULK1_AKT1 | 0.00010 |
| 4 | DRAH_ULK1_AKT1_CSE1L | 0.00003 |
| 5 | DRAM_E2F1_ULK1_AKT1_CSE1L | 0.00002 |
| 6 | DRAM_E2F1_ULK1_AKT1_ATG12_CSE1L | 0.00002 |
| 7 | DRAM_E2F1_ULK1_AKT1_CSE1L_FRAP1_PRKAA1 | 0.00001 |
| 8 | DRAM_E2F1_ULK1_AKT1_ATG12_CSE1L_FRAP1_PRKAA1 | 0.00001 |

As can be seen from the above Table 10, it can be found that as more markers of the present invention are combined, p-values are lower. This means that the more markers of the present disclosure are combined, the lower p-values, which means higher significance, are shown, which means that the more improved accuracy would be achieved in the estimation for prognosis based on the combinations of the markers.

### Example 6: Cross-validation

Cross-validation was performed for combinations of the markers which were statistically significant in Example 5.

369 patients of liver cancer were randomly divided into two groups (positive group: 185 patients; test group: 184 patients). For the positive group, a baseline which was considered statistically significant was established experimentally according to the same method as in Example 3, and a classification was made into high expression and low expression. With the thus-established baseline fixed, for the test group, the accuracy of estimation was calculated to be the level of p<0.05 or p<0.001 with respect to recurrence, overall survival and disease-free survival.

Representative examples showing the excellent accuracy of prognosis with respect to recurrence, overall survival and disease-free survival are as follows:
Recurrence: AIFM1_AKT1_LC3 (77.3% at the level of p<0.05)
Overall survival: ATG5_DRAM_FAS_XIAP (87.3% at the level of p<0.001)
Disease-free survival: AIFM1_AKT1_LC3 (71.3% at the level of p<0.05)

## Claims

1. A method for estimating the prognosis of overall survival in a liver cancer patient comprising:
step 1, contacting a biological sample harvested from the patient having liver cancer with a composition comprising a substance for specifically detecting the expression level of the gene CBS;
step 2, detecting differences in the expression level of CBS by comparing the treatment result of step 1 with a baseline;
wherein the overall survival from liver cancer is prognosticated, and wherein the expression level of CBS higher than the baseline indicates that the prognosis of overall survival is relatively good.

2. A method for estimating the prognosis of overall survival in a liver cancer patient comprising:
step 1, contacting a biological sample harvested from the patient having liver cancer with a composition comprising a substance for specifically detecting the existing level of the protein encoded by the gene CBS; and
step 2, detecting differences in the existing level of the protein encoded by the gene CBS by comparing the treatment result of step 1 with a baseline;
wherein the overall survival from liver cancer is prognosticated, and wherein the existing level of the protein encoded by the gene CBS higher than the baseline indicates that the prognosis of overall survival is relatively good.

## Patentansprüche

1. Verfahren zum Einschätzen der Prognose des Gesamt-Überlebens bei einem Leber-Krebs-Patienten, umfassend:
Schritt 1, In-Kontakt-Bringen einer biologischen Probe, die dem Patienten mit Leber-Krebs entnommen wurde, mit einer Zusammensetzung, umfassend eine Substanz zum spezifischen Nachweis des Expressions-Grads von dem Gen CBS, und;
Schritt 2, Nachweisen von Unterschieden in dem Expressions-Grad von CBS durch Vergleichen des Behandlungs-Ergebnisses von Schritt 1 mit einer Grundlinie;
wobei das Gesamt-Überleben von Leber-Krebs prognostiziert wird, und wobei der Expressions-Grad von CBS höher als die Grundlinie anzeigt, dass die Prognose des Gesamt-Überlebens relativ gut ist.

2. Verfahren zum Einschätzen der Prognose des Gesamt-Überlebens bei einem Leber-Krebs-Patienten, umfassend:
Schritt 1, In-Kontakt-Bringen einer biologischen Probe, die dem Patienten mit Leber-Krebs entnommen wurde, mit einer Zusammensetzung, umfassend eine Substanz zum spezifischen Nachweis der vorliegenden Menge von dem Protein, das durch das Gen CBS kodiert wird; und
Schritt 2, Nachweisen von Unterschieden in der vorliegenden Menge von dem Protein, das durch das Gen CBS kodiert wird, durch Vergleichen des Behandlungs-Ergebnisses von Schritt 1 mit einer Grundlinie;
wobei das Gesamt-Überleben von Leber-Krebs prognostiziert wird, und wobei die vorliegende Menge von dem Protein, das durch das Gen CBS kodiert wird, höher als die Grundlinie anzeigt, dass die Prognose des Gesamt-Überlebens relativ gut ist.

## Revendications

1. Procédé pour estimer le pronostic de survie globale d'un patient ayant un cancer du foie, comprenant :
étape 1, la mise en contact d'un échantillon biologique, récolté auprès du patient ayant un cancer du foie, avec une composition comprenant une substance pour détecter spécifiquement le niveau d'expression du gène CBS ; et
étape 2, la détection de différences du niveau d'expression de CBS par comparaison du résultat du traitement de l'étape 1 avec une valeur de base ;
dans lequel la survie globale avec un cancer du foie est pronostiquée, et dans lequel un niveau d'expression de CBS supérieur à la valeur de base indique que le pronostic de survie globale est relativement bon.

2. Procédé pour estimer le pronostic de survie globale d'un patient ayant un cancer du foie, comprenant :
étape 1, la mise en contact d'un échantillon biologique, récolté auprès du patient ayant un cancer du foie, avec une composition comprenant une substance pour détecter spécifiquement le niveau existant de la protéine codée par le gène CBS ; et
étape 2, la détection de différences du niveau existant de la protéine codée par le gène CBS par comparaison du résultat du traitement de l'étape 1 avec une valeur de base ;
dans lequel la survie globale avec un cancer du foie est pronostiquée, et dans lequel le niveau existant de la protéine codée par le gène CBS supérieur à la valeur de base indique que le pronostic de survie globale est relativement bon.
